# NEW EUROPEAN PATENT SPECIFICATION

(11) **EP 2 967 015 B2**
(45) Date of publication and mention of the opposition decision: **16.09.2026**
(45) Mention of the grant of the patent: 07.06.2023
(21) Application number: 14718815.5
(22) Date of filing: 11.03.2014
(51) Int. Cl.: A01K 67/027, C07K 14/74

(54) **TRANSGENIC MICE EXPRESSING CHIMERIC MAJOR HISTOCOMPATIBILITY COMPLES (MHC) CLASS II MOLECULES**
TRANSGENE MÄUSE, DIE CHIMÄRE MHC-KLASSE-II-MOLEKÜLE EXPRIMIEREN
SOURIS TRANSGÉNIQUES EXPRIMANTS DES MOLÉCULES DU COMPLEXE MAJEUR D'HISTOCOMPATIBILITÉ (CMH) DE CLASSE II CHIMÉRIQUES

(30) Priority: 11.03.2013 US 201313793935
(43) Date of publication of application: 20.01.2016
(60) Divisional application: 23175859.0 / 4 249 507; 26189923.1 / 4 796 560
(73) Proprietor: Regeneron Pharmaceuticals, Inc., Tarrytown, NY 10591 (US)
(72) Inventor: MACDONALD, Lynn, Tarrytown, NY 10591 (US); MURPHY, Andrew J., Tarrytown, NY 10591 (US); TU, Naxin, Tarrytown, NY 10591 (US); GURER, Cagan, Chappaqua, NY 10514 (US); VORONINA, Vera, Sleepy Hollow, NY 10591 (US); STEVENS, Sean, Del Mar, CA 92014 (US)
(74) Representative: Mewburn Ellis LLP
(86) International application number: PCT/US2014/023068
(87) International publication number: WO 2014/164638

(56) References cited:
- WO-A1-2013/063340
- WO-A1-2014/130667
- WO-A2-2005/004592
- US-A1- 2007 209 083
- ITO K ET AL: "HLA-DR4-IE CHIMERIC CLASS II TRANSGENIC, MURINE CLASS II-DEFICIENT MICE ARE SUSCEPTIBLE TO EXPERIMENTAL ALLERGIC ENCEPHALOMYELITIS", THE JOURNAL OF EXPERIMENTAL MEDICINE, ROCKEFELLER UNIVERSITY PRESS, US, vol. 183, no. 6, 1 June 1996 (1996-06-01), pages 2635 - 2644, XP000994711, ISSN: 0022-1007, DOI: 10.1084/JEM.183.6.2635
- A. WOODS ET AL: "Human major histocompatibility complex class II-restricted T cell responses in transgenic mice", JOURNAL OF EXPERIMENTAL MEDICINE, vol. 180, no. 1, 1 July 1994 (1994-07-01), pages 173 - 181, XP055127351, ISSN: 0022-1007, DOI: 10.1084/jem.180.1.173

## Description

### CROSS-REFERENCE TO RELATED APPLICATION

This application claims the benefit of U.S. Application No. 13/793,935, filed March 11, 2013.

### FIELD OF THE INVENTION

Present invention relates to a rodent (e.g., a mouse or a rat) that is genetically engineered to express a humanized Major Histocompatibility Complex (MHC) class II protein, as well as embryos, tissues, and cells expressing the same. The invention further relates to methods for making a genetically modified rodent that expresses a humanized MHC II protein. Also disclosed are methods for using rodents, cells, and tissues that express a humanized MHC class II protein for identifying peptides that activate lymphocytes and engage T cells, and for developing human vaccines and other therapeutics.

### BACKGROUND OF THE INVENTION

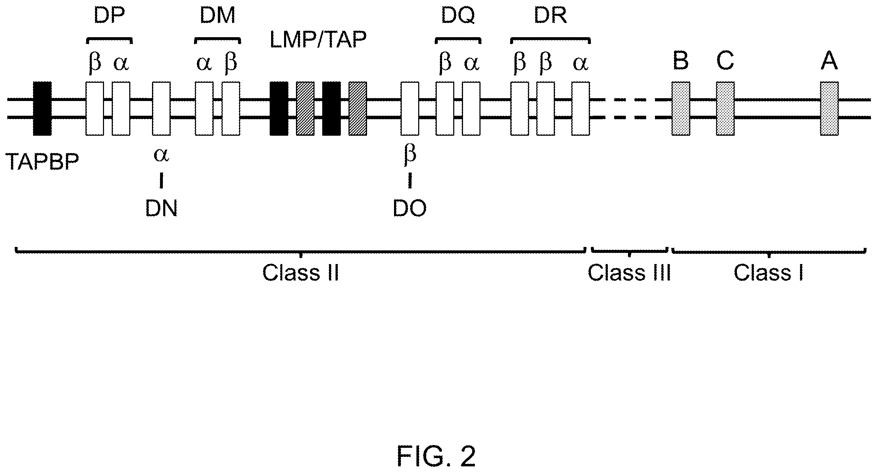

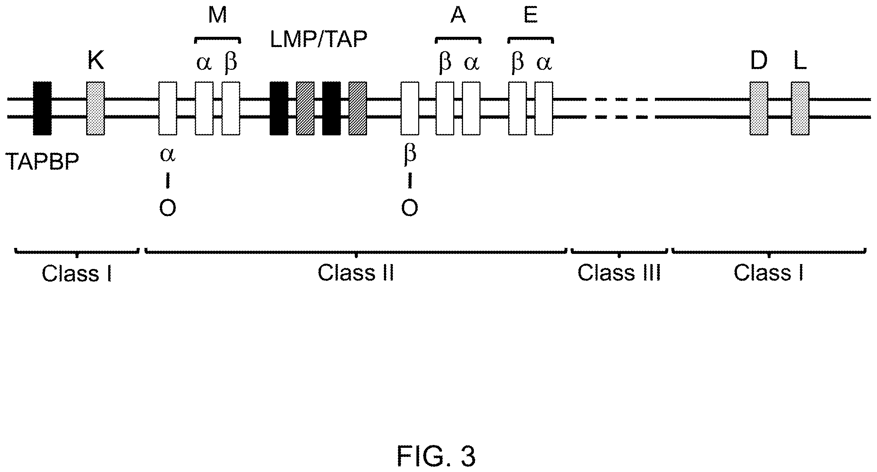

In the adaptive immune response, foreign antigens are recognized by receptor molecules on B lymphocytes (e.g., immunoglobulins) and T lymphocytes (e.g., T cell receptor or TCR). These foreign antigens are presented on the surface of cells as peptide fragments by specialized proteins, generically referred to as major histocompatibility complex (MHC) molecules. MHC molecules are encoded by multiple loci that are found as a linked cluster of genes that spans about 4 Mb. In mice, the MHC genes are found on chromosome 17, and for historical reasons are referred to as the histocompatibility 2 (H-2) genes. In humans, the genes are found on chromosome 6 and are called human leukocyte antigen (HLA) genes. The loci in mice and humans are polygenic; they include three highly polymorphic classes of MHC genes (class I, II and III) that exhibit similar organization in human and murine genomes (see FIG. 2 and FIG. 3, respectively).

MHC loci exhibit the highest polymorphism in the genome; some genes are represented by >300 alleles (e.g., human HLA-DRβ and human HLA-B). All class I and II MHC genes can present peptide fragments, but each gene expresses a protein with different binding characteristics, reflecting polymorphisms and allelic variants. Any given individual has a unique range of peptide fragments that can be presented on the cell surface to B and T cells in the course of an immune response.

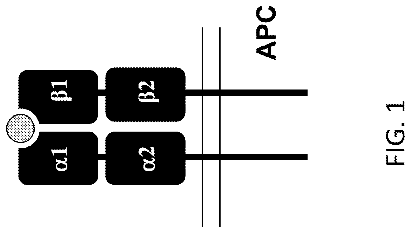

Both humans and mice have class II MHC genes (see FIGs. 2 and 3). In humans, the classical MHC II genes are termed HLA-DP, HLA-DQ, and HLA-DR, whereas in mice they are H-2A and H-2E (often abbreviated as I-A and I-E, respectively). Additional proteins encoded by genes in the MHC II locus, HLA-DM and HLA-DO in humans, and H-2M and H-2O in mice, are not found on the cell surface, but reside in the endocytic compartment and ensure proper loading of MHC II molecules with peptides. Class II molecules consist of two polypeptide chains: α chain and β chain. The extracellular portion of the α chain contains two extracellular domains, α1 and α2; and the extracellular portion of the β chain also contains two extracellular domains, β1 and β2 (see FIG. 1). The α and the β chains are non-covalently associated with each other.

MHC class II molecules are expressed on antigen-presenting cells (APCs), e.g., B cells, macrophages, dendritic cells, endothelial cells during a course of inflammation, etc. MHC II molecules expressed on the surface of APCs typically present antigens generated in intracellular vesicles to CD4+ T cells. In order to participate in CD4+ T cell engagement, the MHC class II complex with the antigen of interest must be sufficiently stable to survive long enough to engage a CD4+ T cell. When a CD4+ T helper cell is engaged by a foreign peptide/MHC II complex on the surface of APC, the T cell is activated to release cytokines that assist in immune response to the invader.

Not all antigens will provoke T cell activation due to tolerance mechanisms. However, in some diseases (e.g., cancer, autoimmune diseases) peptides derived from self-proteins become the target of the cellular component of the immune system, which results in destruction of cells presenting such peptides. There has been significant advancement in recognizing antigens that are clinically significant (e.g., antigens associated with various types of cancer). However, in order to improve identification and selection of peptides that will provoke a suitable response in a human T cell, in particular for peptides of clinically significant antigens, there remains a need for *in vivo* and *in vitro* systems that mimic aspects of human immune system. Thus, there is a need for biological systems (e.g., genetically modified non-human animals and cells) that can display components of a human immune system.

### SUMMARY OF THE INVENTION

A biological system for generating or identifying peptides that associate with human MHC class II proteins and chimeras thereof, and bind to CD4+ T cells, is provided. Rodents comprising rodent cells that express humanized molecules that function in the cellular immune response are provided. Humanized rodent loci that encode humanized MHC II proteins are also disclosed. Humanized rodent cells that express humanized MHC molecules are also disclosed. *In vivo* and *in vitro* systems are disclosed that comprise humanized rodent cells, wherein the rodent cells express one or more humanized immune system molecules.

Disclosed herein is a rodent (e.g., a mouse or a rat) comprising in its genome a nucleotide sequence encoding a humanized MHC II complex, wherein a human portion of the humanized MHC II complex comprises an extracellular domain of a human MHC II complex, e.g., a humanized MHC II α extracellular domain and a humanized MHC II β extracellular domain.

Disclosed herein is a rodent comprising at an endogenous MHC II α gene locus a nucleotide sequence encoding a chimeric human/rodent MHC II α polypeptide. In one example, a human portion of such chimeric human/rodent MHC II α polypeptide comprises a human MHC II α extracellular domain. In the present invention, the rodent expresses a functional MHC II complex on a surface of a cell of the animal. In the present invention, the human MHC II α extracellular domain in the animal comprises human MHC II α1 and α2 domains and a rodent portion of the chimeric human/rodent MHC II α polypeptide comprises transmembrane and cytoplasmic domains of an endogenous rodent MHC II α polypeptide. In one embodiment, the nucleotide sequence encoding a chimeric human/rodent MHC II α polypeptide is operably linked to (e.g., is expressed under regulatory control of) endogenous rodent MHC II α promoter and regulatory elements. In the present invention, the human portion of the chimeric polypeptide is derived from a human HLA class II protein selected from the group consisting of HLA-DR and HLA-DQ, according to the claims, wherein (i) the HLA-DR α polypeptide is an HLA-DR2 α polypeptide and the HLA-DR β polypeptide is an HLA-DR2 β polypeptide, or (ii) the HLA-DR α polypeptide is an HLA-DR4 α polypeptide, the HLA-DR β polypeptide is an HLA-DR4 β polypeptide, the HLA-DQ α polypeptide is an HLA-DQ2 or HLA-DQ8 α polypeptide and the HLA-DQ β polypeptide is an HLA-DQ2 or HLA-DQ8 β. The animal is a rodent, e.g., a mouse. The rodent comprises at an endogenous MHC II α gene locus a nucleotide sequence encoding a chimeric human/rodent MHC II α polypeptide and further comprises at an endogenous MHC II β gene locus a nucleotide sequence encoding a chimeric human/rodent MHC II β polypeptide. Also disclosed herein is a method of making a genetically modified rodent comprising at an endogenous MHC II α gene locus a nucleotide sequence encoding a chimeric human/rodent MHC II α polypeptide. Such a method may comprise replacing at an endogenous MHC II α gene locus a nucleotide sequence encoding an endogenous rodent MHC II α polypeptide with a nucleotide sequence encoding a chimeric human/rodent MHC II α polypeptide.

Also disclosed herein is a rodent comprising at an endogenous MHC II β gene locus a nucleotide sequence encoding a chimeric human/rodent MHC II β polypeptide. In one example, a human portion of such chimeric human/rodent MHC II β polypeptide comprises a human MHC II β extracellular domain. In the present invention, the rodent expresses a functional MHC II complex on a surface of a cell of the animal. The human MHC II β extracellular domain in the animal comprises human MHC II β1 and β2 domains and the rodent portion of the chimeric human/rodent MHC II β polypeptide comprises transmembrane and cytoplasmic domains of an endogenous rodent MHC II β polypeptide. In one embodiment, the nucleotide sequence encoding a chimeric human/rodent MHC II β polypeptide is operably linked to (e.g., is expressed under regulatory control of) endogenous rodent MHC II β promoter and regulatory elements. In the present invention, the human portion of the chimeric polypeptide is derived from a human HLA class II protein selected from the group consisting of HLA-DR and HLA-DQ, according to the claims, wherein (i) the HLA-DR α polypeptide is an HLA-DR2 α polypeptide and the HLA-DR β polypeptide is an HLA-DR2 β polypeptide, or (ii) the HLA-DR α polypeptide is an HLA-DR4 α polypeptide, the HLA-DR β polypeptide is an HLA-DR4 β polypeptide, the HLA-DQ α polypeptide is an HLA-DQ2 or HLA-DQ8 α polypeptide and the HLA-DQ β polypeptide is an HLA-DQ2 or HLA-DQ8 β polypeptide. The animal is a rodent, e.g., a mouse. The rodent comprises at an endogenous MHC II β gene locus a nucleotide sequence encoding a chimeric human/rodent MHC II β polypeptide and further comprises at an endogenous MHC II α gene locus a nucleotide sequence encoding a chimeric human/rodent MHC II α polypeptide. Also disclosed herein is a method of making a genetically modified rodent comprising at an endogenous MHC II β gene locus a nucleotide sequence encoding a chimeric human/rodent MHC II β polypeptide. Such method may comprise replacing at an endogenous MHC II β gene locus a nucleotide sequence encoding an endogenous rodent MHC II β polypeptide with a nucleotide sequence encoding a chimeric human/rodent MHC II β polypeptide.

Disclosed herein is a rodent comprising at an endogenous MHC II gene locus a first nucleotide sequence encoding a chimeric human/rodent MHC II α polypeptide and a second nucleotide sequence encoding a chimeric human/rodent MHC II β polypeptide, wherein a human portion of the chimeric human/rodent MHC II α polypeptide comprises a human MHC II α extracellular domain and a human portion of the chimeric human/rodent MHC II β polypeptide comprises a human MHC II β extracellular domain. In the present invention the chimeric human/rodent MHC II α and β polypeptides form a functional chimeric MHC II complex (e.g., human/rodent MHC II complex) on a surface of a cell. In the present invention, the human MHC II α extracellular domain comprises human α1 and α2 domains of human MHC II. In the present invention, the human MHC II β extracellular domain comprises human β1 and β2 domains of human MHC II. In various aspects, the first nucleotide sequence is expressed under regulatory control of (e.g., is operably linked to) endogenous rodent MHC II α promoter and regulatory elements. In various aspects, the second nucleotide sequence is expressed under regulatory control of (e.g., is operably linked to) endogenous rodent MHC II β promoter and regulatory elements. A rodent portion of the chimeric human/rodent MHC II α polypeptide comprises transmembrane and cytoplasmic domains of an endogenous rodent MHC II α polypeptide. A rodent portion of the chimeric human/rodent MHC II β polypeptide comprises transmembrane and cytoplasmic domains of an endogenous rodent MHC II β polypeptide.

In the present invention, the animal is a rodent, and the human portions of the chimeric human/rodent MHC II α and β polypeptides comprise human sequences derived from HLA class II protein selected from the group consisting of HLA-DR and HLA-DQ wherein (i) the HLA-DR α polypeptide is an HLA-DR2 α polypeptide and the HLA-DR β polypeptide is an HLA-DR2 β polypeptide or (ii) the H. In some embodiments of the invention, wherein the human portions of the chimeric human/rodent MHC II α and β sequences are derived from a human HLA-DR4 sequence; the nucleotide sequence encoding the MHC II α extracellular domain is derived from a sequence of an HLA-DRα*01 gene, and the nucleotide sequence encoding the MHC II β extracellular domain is derived from a sequence of an HLA-DRβ 1 *04 gene. In some embodiments of the invention, wherein the human portions of the chimeric human/rodent MHC II α and β sequences are derived from a human HLA-DR2 sequence, the nucleotide sequence encoding the MHC II α extracellular domain is derived from a sequence of an HLA-DRα1*01 gene, and the nucleotide sequence encoding the MHC II β extracellular domain is derived from a sequence of an HLA-DRβ1*02 gene (e.g., HLA-DRβ1*02(1501) gene). In some embodiments of the invention, wherein the human portions of the chimeric human/rodent MHC II α and β sequences are derived from a human HLA-DQ2 sequence; the nucleotide sequence encoding the MHC II α extracellular domain is derived from a sequence of an HLA-DQα1 *05 gene (e.g., HLA-DQA1*1501 gene), and the nucleotide sequence encoding the MHC II β extracellular domain is derived from a sequence of an HLA-DQβ1*02 gene. In some embodiments of the invention, wherein the human portions of the chimeric human/rodent MHC II α and β sequences are derived from a human HLA-DQ8 sequence; the nucleotide sequence encoding the MHC II α extracellular domain is derived from a sequence of an HLA-DQα1*0301 gene, and the nucleotide sequence encoding the MHC II β extracellular domain is derived from a sequence of an HLA-DQβ1*0302 gene.

In various embodiments of the invention, the first and the second nucleotide sequences are located on the same chromosome. In some examples the animal comprises two copies of the MHC II locus containing the first and the second nucleotide sequences, while in other examples the animal comprises one copy of the MHC II locus containing the first and the second nucleotide sequences. Thus, the animal may be homozygous or heterozygous for the MHC II locus containing the first and the second nucleotide sequences. In one example the chimeric MHC II described herein is in the germline of a rodent.

In some embodiments, the chimeric MHC II α polypeptide and/or the chimeric MHC II β polypeptide is operably linked to a rodent leader sequence.

In the present invention, the genetically engineered animal is a rodent. In one embodiment, the rodent is selected from the group consisting of a mouse and a rat. Thus, in some embodiments, rodent sequences of the chimeric MHC II α and β genes are derived from nucleotide sequences encoding mouse MHC II protein, e.g., mouse H-2A or H-2E protein. In one embodiment, the rodent (e.g., the mouse or the rat) of the invention does not express functional endogenous MHC II polypeptides from their endogenous loci. In one embodiment, wherein the rodent is a mouse, the mouse does not express functional endogenous H-2E and H-2A polypeptides from their endogenous loci.

Thus, in some embodiments, a mouse is provided comprising at an endogenous mouse MHC II locus a first nucleotide sequence encoding a chimeric human/mouse MHC II α polypeptide and a second nucleotide sequence encoding a chimeric human/mouse MHC II β polypeptide, wherein (i) a human portion of the chimeric MHC II α polypeptide comprises an extracellular domain derived from an α polypeptide of a human HLA class II protein from HLA-DR2, and a human portion of the chimeric human/mouse MHC II β polypeptide comprises a human portion of the chimeric MHC II α polypeptide comprises an extracellular domain derived from an α polypeptide of a human HLA class II protein selected from HLA-DR4, HLA-DQ2, and HLA-DQ8 and a human portion of the chimeric human/mouse MHC II β polypeptide comprises an extracellular domain derived from a β polypeptide of a human HLA class II protein selected from HLA-DR4, HLA-DQ2, and HLA-DQ8, wherein a mouse portion of the chimeric MHC II α polypeptide comprises transmembrane and cytoplasmic domains of a mouse H-2A or H-2E α chain and a mouse portion of the chimeric MHC II β polypeptide comprises transmembrane and cytoplasmic domains of a mouse H-2A or H-2E β chain, and wherein the mouse expresses a functional chimeric HLA class II complex. In one embodiment, the functional chimeric HLA class II complex is HLA-DR4/H-2E. In another embodiment, the functional chimeric HLA class II complex is HLA-DR2/H-2E. In another embodiment, the functional chimeric HLA class II complex is HLA-DQ2/H-2A. In yet another embodiment, the functional chimeric HLA class II complex is HLA-DQ8/H-2A. The extracellular domain of the chimeric MHC II α polypeptide comprises human α1 and α2 domains and the extracellular domain of the chimeric MHC II β polypeptide comprises human β1 and β2 domains. In some embodiments, the first nucleotide sequence is expressed under regulatory control of (e.g., is operably linked to) endogenous mouse MHC II α promoter and regulatory elements, and the second nucleotide sequence is expressed under regulatory control of (e.g., is operably linked to) endogenous mouse MHC II β promoter and regulatory elements. In various embodiments, the mouse does not express functional endogenous MHC II polypeptides, e.g., H-2E and H-2A polypeptides, from their endogenous loci. In some aspects, the mouse comprises two copies of the MHC II locus containing the first and the second nucleotide sequences, while in other aspects, the mouse comprises one copy of the MHC II locus containing the first and the second nucleotide sequences.

Provided herein is a mouse that comprises in its genome, e.g., at its endogenous MHC II locus, two or more, e.g., two, three, four, nucleotide sequence(s) encoding a chimeric human/mouse MHC II complex(es). Each complex comprises an MHC II α and an MHC II β polypeptides, and a human portion of the chimeric MHC II α and MHC II β polypeptides comprises an extracellular domain of a human MHC II α and MHC II β polypeptides, respectively, while a mouse portion comprises transmembrane and cytoplasmic domains of a mouse MHC II α and MHC II β polypeptides, respectively. Thus, the mouse may express two or more, e.g., two, three, four, chimeric human/mouse MHC II complex(es).

Methods of making genetically engineered rodents (e.g., mice or rats) as described herein are also provided. In various embodiments, rodents (e.g., mice or rats) of the invention are made by replacing endogenous MHC II sequences with nucleotide sequences encoding chimeric human/rodent (e.g., human/mouse) MHC II α and β polypeptides. In one example, the invention provides a method of modifying an MHC II locus of a rodent (e.g., a mouse or a rat) to express a chimeric human/rodent MHC II complex comprising replacing at the endogenous rodent MHC II locus a nucleotide sequence encoding a rodent MHC II complex with a nucleotide sequence encoding a chimeric human/rodent MHC II complex. In one example of the method, the nucleotide sequence encoding the chimeric human/rodent MHC II complex comprises a first nucleotide sequence encoding an extracellular domain of a human MHC II α chain and transmembrane and cytoplasmic domains of a rodent MHC II α chain and a second nucleotide sequence encoding an extracellular domain of a human MHC II β chain and transmembrane and cytoplasmic domains of a rodent MHC II β chain. In some examples, a rodent portion of the chimeric MHC II complex is derived from a mouse H-2E protein, and a human portion is derived from a human HLA-DR4 protein. In some examples, a rodent portion of the chimeric MHC II complex is derived from a mouse H-2E protein, and a human portion is derived from a human HLA-DR2 protein. In some examples, a rodent portion of the chimeric MHC II complex is derived from a mouse H-2A protein, and a human portion is derived from a human HLA-DQ2 protein. In yet other aspects, a rodent portion of the chimeric MHC II complex is derived from a mouse H-2A protein, and a human portion is derived from a human HLA-DQ8 protein. In some embodiments, the replacement of the endogenous MHC II loci described herein is made in a single ES cell, and the single ES cell is introduced into a rodent (e.g., mouse or rat) embryo to make a genetically modified rodent (e.g., mouse or rat).

Also disclosed herein is a rodent chimeric MHC II locus encoding a chimeric human/rodent MHC II complex, comprising a first nucleotide sequence encoding an extracellular domain of a human MHC II α chain and transmembrane and cytoplasmic domains of a rodent MHC II α chain and a second nucleotide sequence encoding an extracellular domain of a human MHC II β chain and transmembrane and cytoplasmic domains of a rodent MHC II β chain. In one example, the chimeric MHC II locus is at an endogenous MHC II position in a genome of a rodent. In one example, the chimeric MHC II locus expresses a chimeric human/rodent (e.g., human/mouse or human/rat) MHC II complex. In one example, the human MHC II is selected from HLA-DQ and HLA-DR, according to the claims, wherein (i) the HLA-DR α polypeptide is an HLA-DR2 α polypeptide and the HLA-DR β polypeptide is an HLA-DR2 β polypeptide, or (ii) the HLA-DR α polypeptide is an HLA-DR4 α polypeptide, the HLA-DR β polypeptide is an HLA-DR4 β polypeptide, the HLA-DQ α polypeptide is an HLA-DQ2 or HLA-DQ8 α polypeptide and the HLA-DQ β polypeptide is an HLA-DQ2 or HLA-DQ8 β polypeptide). In one example, the rodent MHC II is mouse MHC II selected from H-2A and H-2E. In one example, the chimeric MHC II locus is obtainable by any methods described herein for generating genetically modified rodents (e.g., mice or rats).

Also disclosed herein are cells, e.g., isolated antigen-presenting cells, derived from the rodents (e.g., mice or rats) described herein. Tissues and embryos derived from the rodents described herein are also disclosed

### BRIEF DESCRIPTION OF THE DRAWINGS

**FIG. 1** is a schematic drawing of the MHC II class molecule expressed on the surface of an antigen presenting cell (APC), containing four domains: α1, α2, β1, and β2. The gray circle represents a peptide bound in the peptide-binding cleft.
**FIG. 2** is a schematic representation (not to scale) of the relative genomic structure of the human HLA, showing class I, II and III genes.
**FIG. 3** is a schematic representation (not to scale) of the relative genomic structure of the mouse MHC, showing class I, II and III genes.

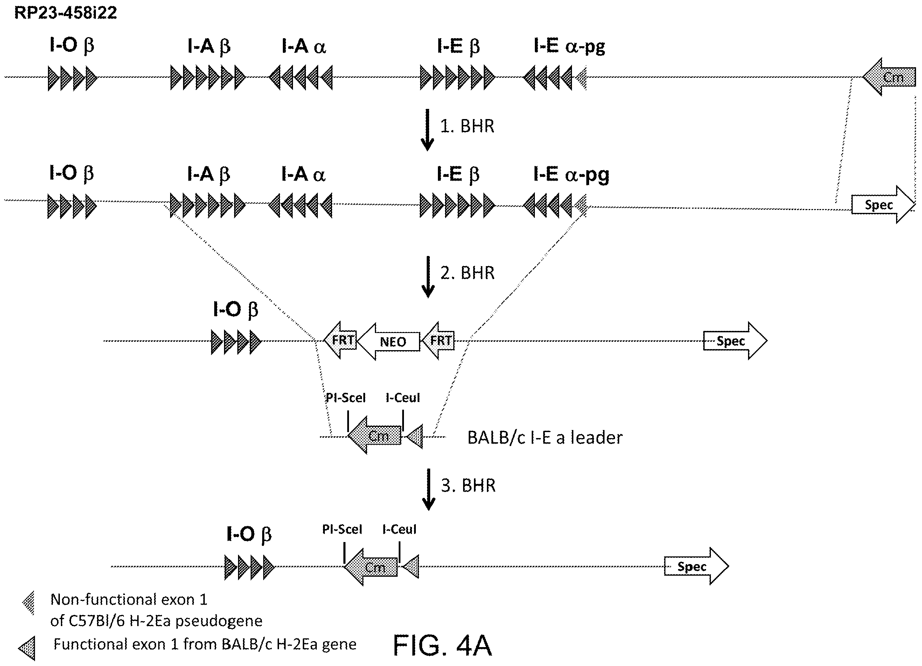

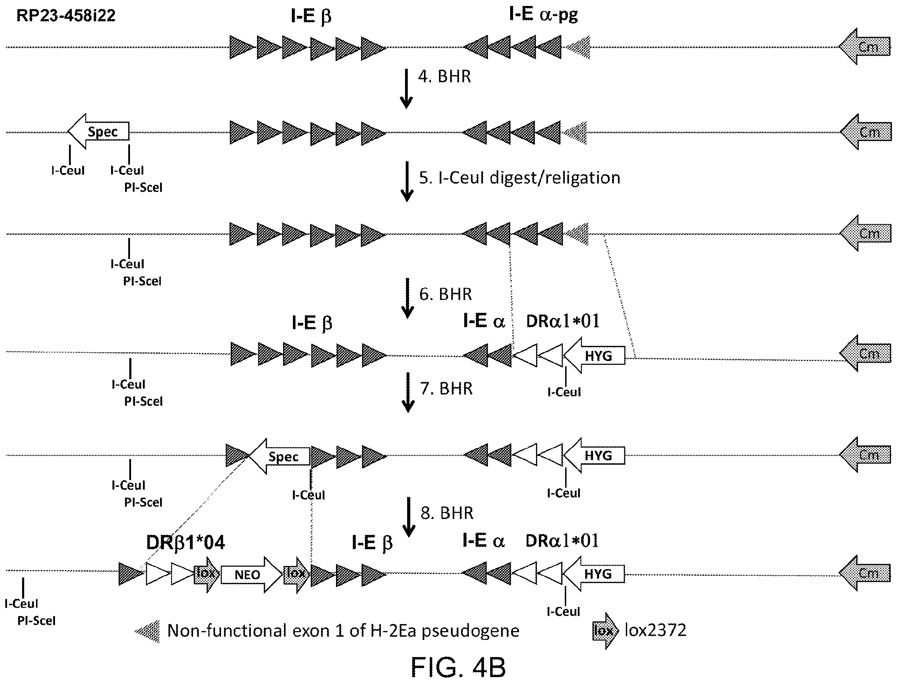

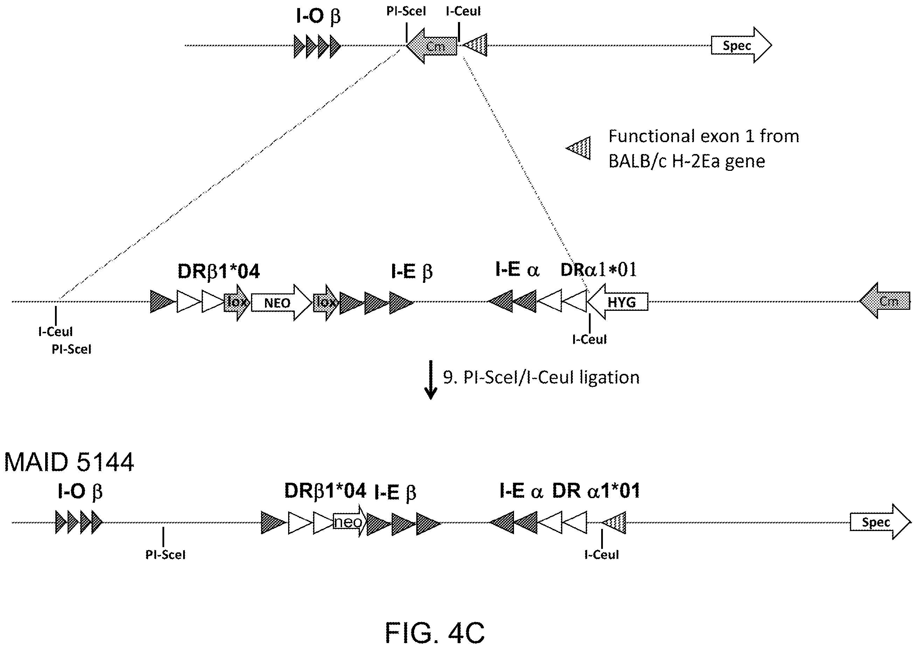

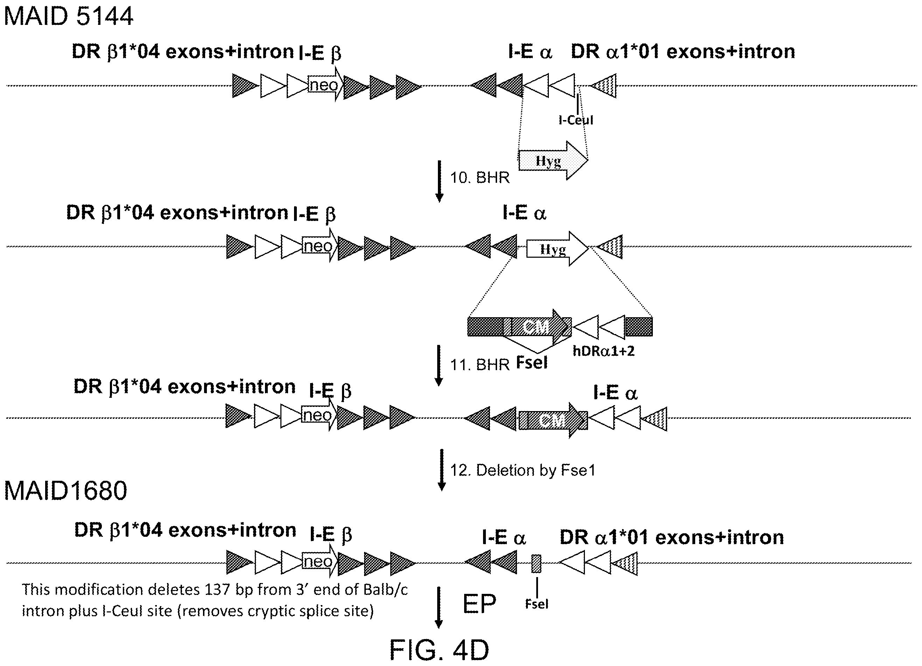

**FIG. 4** **(A-D)** is a schematic illustration (not to scale) of the strategy for generating a targeting vector comprising humanized I-E β and I-E α (i.e., H-2Eβ/HLA-DRβ1^{∗}04 and H-2Eα/HLA-DRα^{∗}01 chimera, respectively). In FIG. 4C, the final humanized MHC II sequence from FIG. 4B is ligated between PI-SceI and I-CeuI restriction sites of the final construct from FIG. 4A, to generate a construct comprising humanized MHC II and exon 1 of I-Eα from BALB/c. Pg=pseudogene; BHR= bacterial homologous recombination; CM=chloramphenicol; spec=spectinomycin; hyg=hygromycin; neo=neomycin; EP=electroporation. Triangles represent exons, filled triangles represent mouse exons from C57BL/6 mouse (with the exception of hashed triangles, which represent exon 1 of I-Eα from BALB/c mouse) and open triangles represent human exons.
**FIG. 5** shows a schematic illustration, not to scale, of MHC class II I-E and I-A genes, showing knockout of the mouse locus using a hygromycin cassette, followed by introduction of a vector comprising a humanized I-E β and I-E α (i.e., H-2Eβ/HLA-DRβ1^{∗}04 and H-2Eα/HLA-DRα^{∗}01 chimera, respectively). Open triangles represent human exons; filled triangles represent mouse exons. Probes used for genotyping are encircled.
**FIG. 6** shows a schematic illustration, not to scale, of Cre-mediated removal of the neomycin cassette of FIG. 5. Open triangles represent human exons; filled triangles represent mouse exons. Top two strands represent MHC II loci in humanized MHC II heterozygous mouse harboring a neomycin selection cassette, and bottom two strands represent MHC II loci in humanized MHC II heterozygous mouse with neomycin cassette removed.

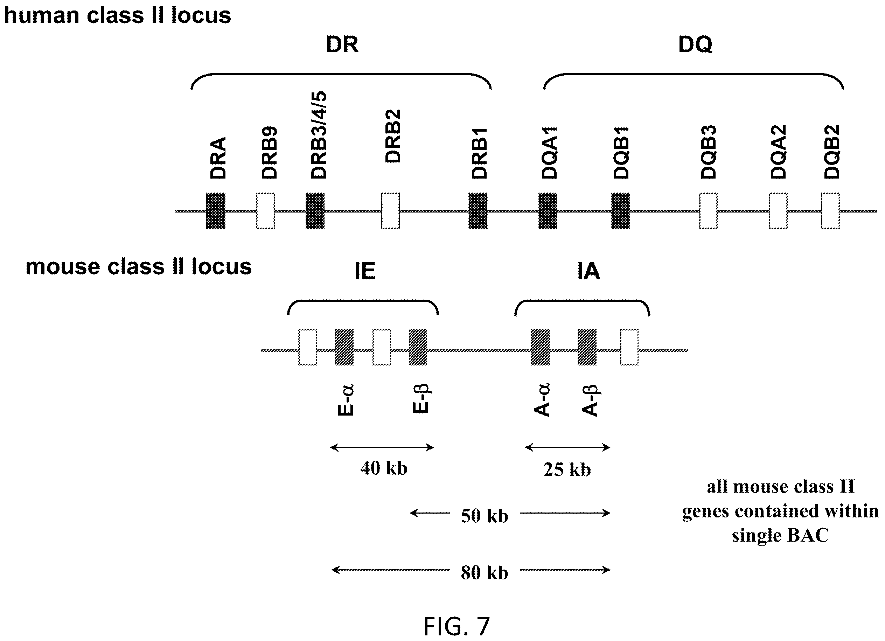

**FIG. 7** shows a schematic comparative illustration, not to scale, of mouse and human class II loci. Class II genes are represented by boxes. Relative sizes (kb) of various nucleic acid fragments are included.

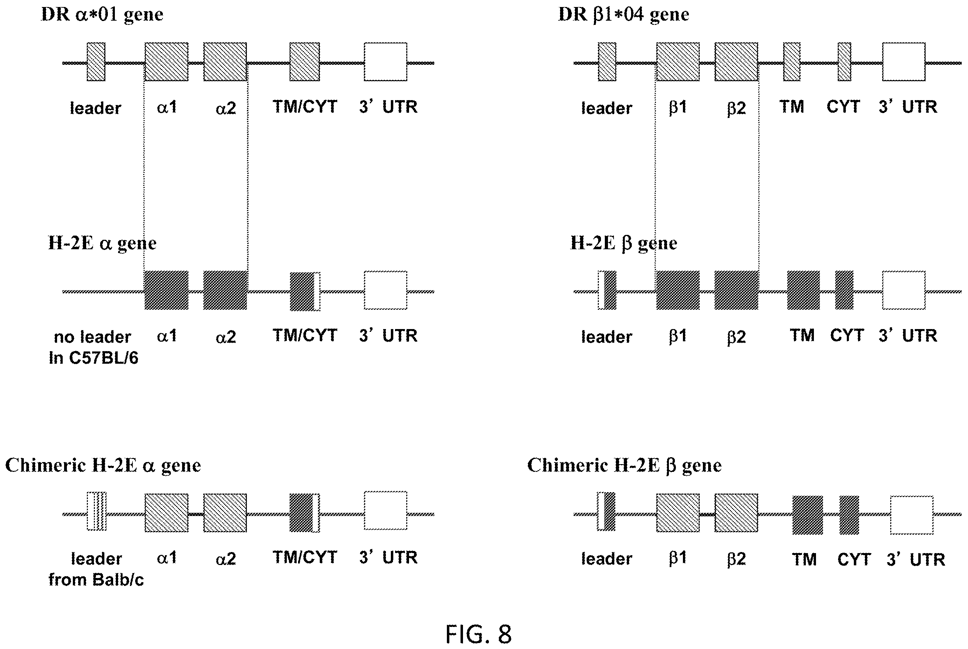

**FIG. 8****,** at left panel, is a schematic illustration (not to scale) of humanization strategy for the MHC II α chain; in particular, the figure shows a replacement of α1 and α2 domains, encoded by exons 2 and 3 of MHC II α gene, while retaining mouse transmembrane and cytoplasmic tail sequences. In the humanized locus, the MHC II α leader sequence is derived from the mouse BALB/c strain. The right panel illustrates humanization of the MHC II β chain; in particular, the figure shows a replacement of β1 and β2 domains, encoded by exons 2 and 3 of MHC II β gene, while retaining the mouse leader and mouse transmembrane and cytoplasmic tail sequences. Top row are all human sequences; middle row are all mouse sequences; bottom row are all humanized sequences, with exons 2 and 3 derived from human HLA-DR genes.
**FIG. 9** shows FACS analysis with anti-HLA-DR antibody of B cells from a mouse heterozygous for a chimeric HLA-DR4 (neo cassette removed) in the presence (1681HET + poly(I:C) or absence (1681HET) of poly(I:C), and a wild-type mouse (WT mouse).

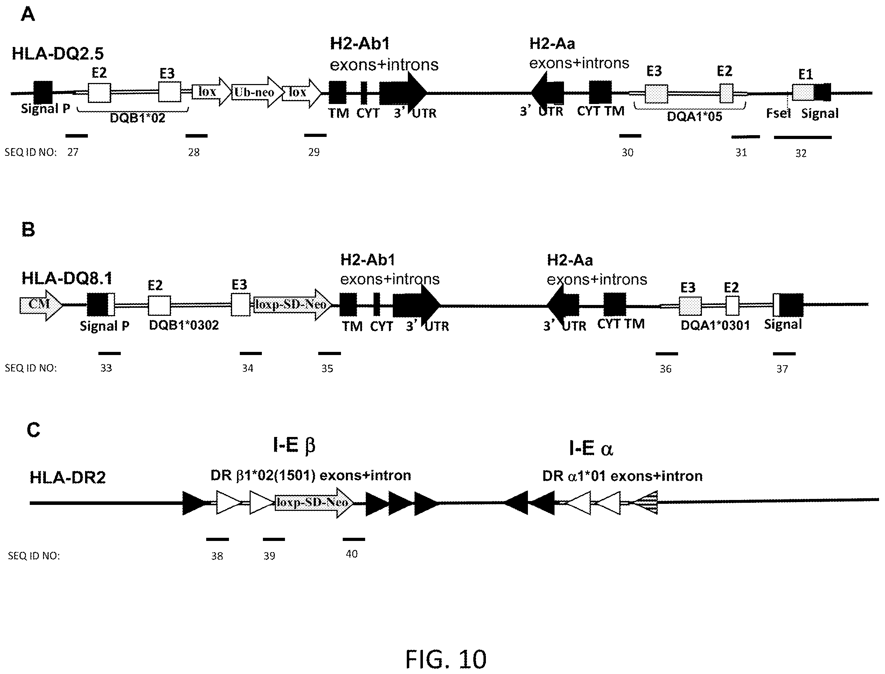

**FIG. 10** is a schematic illustration (not to scale) of the targeting vectors used for generating humanized MHC II genes, specifically, a targeting vector for generating HLA-DQ2.5/H2-A mouse (**FIG. 10A**), a targeting vector for generating HLA-DQ8.1/H-2A mouse (**FIG. 10B**), and a targeting vector for generating HLA-DR2/H-2E mouse (**FIG. 10C**). In the diagrams, unless indicated otherwise (e.g., *lox*P site, etc.), the empty boxes or triangles are sequences of the human exons, the double lines are sequences of the human introns, the filed boxes or triangles are sequences of the mouse exons, single lines are sequences of mouse introns, and the hashed triangle is exon 1 of I-Eα from BALB/c mouse. Location of junctional sequences are indicated below each targeting vector diagram and presented in Table 1 and the Sequence Listing.
**FIG. 11** shows mouse H-2A/H-2E (IA/IE) and human HLA-DQ2.5 expression on CD19+ B cells obtained from WT or heterozygous HLA-DQ2.5/H-2A ("6040Het") mice.

### DETAILED DESCRIPTION OF THE INVENTION

### Definitions

The present invention provides genetically modified rodents (e.g., mice, rats, etc.) that express humanized MHC II polypeptide; and further discloses embryos, cells, and tissues comprising the same; methods of making the same; as well as methods of using the same. Unless defined otherwise, all terms and phrases used herein include the meanings that the terms and phrases have attained in the art, unless the contrary is clearly indicated or clearly apparent from the context in which the term or phrase is used.

The term "conservative," when used to describe a conservative amino acid substitution, includes substitution of an amino acid residue by another amino acid residue having a side chain R group with similar chemical properties (e.g., charge or hydrophobicity). Conservative amino acid substitutions may be achieved by modifying a nucleotide sequence so as to introduce a nucleotide change that will encode the conservative substitution. In general, a conservative amino acid substitution will not substantially change the functional properties of interest of a protein, for example, the ability of MHC II to present a peptide of interest. Examples of groups of amino acids that have side chains with similar chemical properties include aliphatic side chains such as glycine, alanine, valine, leucine, and isoleucine; aliphatic-hydroxyl side chains such as serine and threonine; amide-containing side chains such as asparagine and glutamine; aromatic side chains such as phenylalanine, tyrosine, and tryptophan; basic side chains such as lysine, arginine, and histidine; acidic side chains such as aspartic acid and glutamic acid; and, sulfur-containing side chains such as cysteine and methionine. Conservative amino acids substitution groups include, for example, valine/leucine/isoleucine, phenylalanine/tyrosine, lysine/arginine, alanine/valine, glutamate/aspartate, and asparagine/glutamine. In some embodiments, a conservative amino acid substitution can be a substitution of any native residue in a protein with alanine, as used in, for example, alanine scanning mutagenesis. In some embodiments, a conservative substitution is made that has a positive value in the PAM250 log-likelihood matrix disclosed in Gonnet et al. ((1992) Exhaustive Matching of the Entire Protein Sequence Database, Science 256:1443-45).

In some embodiments, the substitution is a moderately conservative substitution wherein the substitution has a nonnegative value in the PAM250 log-likelihood matrix.

Thus, also encompassed by the invention is a genetically modified rodent whose genome comprises a nucleotide sequence encoding a humanized MHC II polypeptide, wherein the polypeptide comprises conservative amino acid substitutions in the amino acid sequence described herein.

One skilled in the art would understand that in addition to the nucleic acid residues encoding a humanized MHC II polypeptide described herein, due to the degeneracy of the genetic code, other nucleic acids may encode the polypeptide of the invention. Therefore, in addition to a genetically modified rodent that comprises in its genome a nucleotide sequence encoding MHC II polypeptide with conservative amino acid substitutions, a rodent whose genome comprises a nucleotide sequence that differs from that described herein due to the degeneracy of the genetic code is also provided.

The term "identity" when used in connection with sequence includes identity as determined by a number of different algorithms known in the art that can be used to measure nucleotide and/or amino acid sequence identity. In some embodiments described herein, identities are determined using a ClustalW v. 1.83 (slow) alignment employing an open gap penalty of 10.0, an extend gap penalty of 0.1, and using a Gonnet similarity matrix (MacVector^{™} 10.0.2, MacVector Inc., 2008). The length of the sequences compared with respect to identity of sequences will depend upon the particular sequences. In various embodiments, identity is determined by comparing the sequence of a mature protein from its N-terminal to its C-terminal. In various examples when comparing a chimeric human/non-human sequence to a human sequence, the human portion of the chimeric human/non-human sequence (but not the non-human portion) is used in making a comparison for the purpose of ascertaining a level of identity between a human sequence and a human portion of a chimeric human/non-human sequence (e.g., comparing a human ectodomain of a chimeric human/mouse protein to a human ectodomain of a human protein).

The terms "homology" or "homologous" in reference to sequences, e.g., nucleotide or amino acid sequences, means two sequences which, upon optimal alignment and comparison, are identical in at least about 75% of nucleotides or amino acids, e.g., at least about 80% of nucleotides or amino acids, e.g., at least about 90-95% nucleotides or amino acids, e.g., greater than 97% nucleotides or amino acids. One skilled in the art would understand that, for optimal gene targeting, the targeting construct should contain arms homologous to endogenous DNA sequences (i.e., "homology arms"); thus, homologous recombination can occur between the targeting construct and the targeted endogenous sequence.

The term "operably linked" refers to a juxtaposition wherein the components so described are in a relationship permitting them to function in their intended manner. As such, a nucleic acid sequence encoding a protein may be operably linked to regulatory sequences (e.g., promoter, enhancer, silencer sequence, etc.) so as to retain proper transcriptional regulation. In addition, various portions of the chimeric or humanized protein of the invention may be operably linked to retain proper folding, processing, targeting, expression, and other functional properties of the protein in the cell. Unless stated otherwise, various domains of the chimeric or humanized protein of the invention are operably linked to each other.

The terms "MHC II complex," "MHC II protein," or the like, as used herein, include the complex between an MHC II α polypeptide and an MHC II β polypeptide. The term "MHC II α polypeptide" or "MHC II β polypeptide" (or the like), as used herein, includes the MHC II α polypeptide alone or MHC II β polypeptide alone, respectively. Similarly, the terms "HLA-DR4 complex", "HLA-DR4 protein," "H-2E complex," "H-2E" protein," or the like (e.g., similar terms referring to other MHC II alleles), refer to complex between α and β polypeptides. Typically, the terms "human MHC" and "HLA" are used interchangeably.

The term "replacement" in reference to gene replacement refers to placing exogenous genetic material at an endogenous genetic locus, thereby replacing all or a portion of the endogenous gene with an orthologous or homologous nucleic acid sequence. As demonstrated in the Examples below, nucleic acid sequence of endogenous MHC II locus was replaced by a nucleotide sequence comprising sequences encoding portions of human MHC II α and β polypeptides; specifically, encoding the extracellular portions of the MHC II α and β polypeptides.

"Functional" as used herein, e.g., in reference to a functional polypeptide, refers to a polypeptide that retains at least one biological activity normally associated with the native protein. For example, in some embodiments of the invention, a replacement at an endogenous locus (e.g., replacement at an endogenous non-human MHC II locus) results in a locus that fails to express a functional endogenous polypeptide. Likewise, the term "functional" as used herein in reference to functional extracellular domain of a protein, refers to an extracellular domain that retains its functionality, e.g., in the case of MHC II, ability to bind an antigen, ability to bind a T cell co-receptor, etc. In some embodiments of the invention, a replacement at the endogenous MHC locus results in a locus that fails to express an extracellular domain (e.g., a functional extracellular domain) of an endogenous MHC while expressing an extracellular domain (e.g., a functional extracellular domain) of a human MHC.

### Genetically Modified MHC II Animals

In various aspects, the invention generally provides genetically modified rodents that comprise in their genome a nucleotide sequence encoding a humanized MHC II complex; thus, the animals express a humanized MHC II complex (e.g., MHC II α and β polypeptides).

MHC genes are categorized into three classes: class I, class II, and class III, all of which are encoded either on human chromosome 6 or mouse chromosome 17. A schematic of the relative organization of the human and mouse MHC classes is presented in FIGs. 2 and 3, respectively. The majority of MHC genes are polymorphic, in fact they are the most polymorphic genes of the mouse and human genomes. MHC polymorphisms are presumed to be important in providing evolutionary advantage; changes in sequence can result in differences in peptide binding that allow for better antigen presentation. One exception is the human HLA-DRα chain and its mouse homolog, Eα (i.e., H-2Ea), which are monomorphic.

MHC class II complex comprises two non-covalently associated domains: an α chain and a β chain, also referred herein as an α polypeptide and a β polypeptide (FIG. 1). The protein spans the plasma membrane; thus it contains an extracellular domain, a transmembrane domain, and a cytoplasmic domain. The extracellular portion of the α chain includes α1 and α2 domains, and the extracellular portion of the β chain includes β1 and β2 domains. The α1 and β1 domains form a peptide-binding cleft on the cell surface. Due to the three-dimensional confirmation of the peptide-binding cleft of the MHC II complex, there is theoretically no upper limit on the length of the bound antigen, but typically peptides presented by MHC II are between 13 and 17 amino acids in length.

In addition to its interaction with the antigenic peptides, the peptide-binding cleft of the MHC II molecule interacts with invariant chain (Ii) during the processes of MHC II complex formation and peptide acquisition. The α/β MHC II dimers assemble in the endoplasmic reticulum and associate with Ii chain, which is responsible for control of peptide binding and targeting of the MHC II into endocytic pathway. In the endosome, Ii undergoes proteolysis, and a small fragment of Ii, Class II-associated invariant chain peptide (CLIP), remains at the peptide-binding cleft. In the endosome, under control of HLA-DM (in humans), CLIP is exchanged for antigenic peptides.

MHC II interacts with T cell co-receptor CD4 at the hydrophobic crevice at the junction between α2 and β2 domains. Wang and Reinherz (2002) Structural Basis of T Cell Recognition of Peptides Bound to MHC Molecules, Molecular Immunology, 38:1039-49. When CD4 and T cell receptor bind the same MHC II molecule complexed with a peptide, the sensitivity of a T cell to antigen is increased, and it requires 100-fold less antigen for activation. *See,* Janeway's Immunobiology, 7th Ed., Murphy et al. eds., Garland Science, 2008.

Numerous functions have been proposed for transmembrane and cytoplasmic domains of MHC II. In the case of cytoplasmic domain, it has been shown to be important for intracellular signaling, trafficking to the plasma membrane, and ultimately, antigen presentation. For example, it was shown that T cell hybridomas respond poorly to antigen-presenting cells (APCs) transfected with MHC II β chains truncated at the cytoplasmic domain, and induction of B cell differentiation is hampered. *See, e.g.,* Smiley et al. (1996) Truncation of the class II β-chain cytoplasmic domain influences the level of class II/invariant chain-derived peptide complexes, Proc. Natl. Acad. Sci. USA, 93:241-44. Truncation of Class II molecules seems to impair cAMP production. It has been postulated that deletion of the cytoplasmic tail of MHC II affects intracellular trafficking, thus preventing the complex from coming across relevant antigens in the endocytic pathway. Smiley et al. (*supra*) demonstrated that truncation of class II molecules at the cytoplasmic domain reduces the number of CLIP/class II complexes, postulating that this affects the ability of CLIP to effectively regulate antigen presentation.

It has been hypothesized that, since MHC II clustering is important for T cell receptor (TCR) triggering, if MHC II molecules truncated at the cytoplasmic domain were prevented from binding cytoskeleton and thus aggregating, antigen presentation to T cells would be affected. Ostrand-Rosenberg et al. (1991) Abrogation of Tumorigenicity by MHC Class II Antigen Expression Requires the Cytoplasmic Domain of the Class II Molecule, J. Immunol. 147:2419-22. In fact, it was recently shown that HLA-DR truncated at the cytoplasmic domain failed to associate with the cytoskeleton following oligomerization. El Fakhy et al. (2004) Delineation of the HLA-DR Region and the Residues Involved in the Association with the Cytoskeleton, J. Biol. Chem. 279:18472-80. Importantly, actin cytoskeleton is a site of localized signal transduction activity, which can effect antigen presentation. In addition to association with cytoskeleton, recent studies have also shown that up to 20% of all HLA-DR molecules constitutively reside in the lipid rafts of APCs, which are microdomains rich in cholesterol and glycosphingolipids, and that such localization is important for antigen presentation, immune synapse formation, and MHC II-mediated signaling. *See, e.g.,* Dolan et al. (2004) Invariant Chain and the MHC II Cytoplasmic Domains Regulate Localization of MHC Class II Molecules to Lipid Rafts in Tumor Cell-Based Vaccines, J. Immunol. 172:907-14. Dolan et al. suggested that truncation of cytoplasmic domain of MHC II reduces constitutive localization of MHC II to lipid rafts.

In addition, the cytoplasmic domain of MHC II, in particular the β chain, contains a leucine residue that is subject to ubiquitination by ubiquitin ligase, membrane-associated RING-CH I (MARCH I), which controls endocytic trafficking, internalization, and degradation of MHC II; and it has been shown that MARCH-mediated ubiquitination ceases upon dendritic cell maturation resulting in increased levels of MHC II at the plasma membrane. Shin et al. (2006) Surface expression of MHC class II in dendritic cells is controlled by regulated ubiquitination, Nature 444:115-18; De Gassart et al. (2008) MHC class II stabilization at the surface of human dendritic cells is the result of maturation-dependent MARCH I down-regulation, Proc. Natl. Acad. Sci. USA 105:3491-96.

Transmembrane domains of α and β chains of MHC II interact with each other and this interaction is important for proper assembly of class II MHC complex. Cosson and Bonifacino (1992) Role of Transmembrane Domain Interactions in the Assembly of Class II MHC Molecules, Nature 258:659-62. In fact, MHC II molecules in which the transmembrane domains of the α and β chains were replaced by the α chain of IL-2 receptor were retained in the ER and were barely detectable at the cell surface. *Id.* Through mutagenesis studies, conserved Gly residues at the α and β transmembrane domains were found to be responsible for MHC II assembly at the cell surface. *Id.* Thus, both transmembrane and cytoplasmic domains are crucial for the proper function of the MHC II complex.

Disclosed herein is a genetically modified rodent (e.g., mouse, rat, etc.) that comprises in its genome a nucleotide sequence encoding a humanized MHC II complex, e.g., a humanized MHC II α and β polypeptide(s). The rodent comprises in its genome a nucleotide sequence that encodes an MHC II complex that is partially human and partially non-human, e.g., a rodent that expresses a chimeric human/rodent MHC II complex (e.g., a rodent that expresses chimeric human/rodent MHC II α and β polypeptides). In one embodiment, the rodent only expresses the humanized MHC II complex, e.g., a chimeric human/rodent MHC II complex, and does not express an endogenous rodent MHC II complex from an endogenous MHC II locus. In some embodiments, the animal is incapable of expressing any endogenous rodent MHC II complex from an endogenous MHC II locus, but only expresses the humanized MHC II complex. In other embodiments, the animal retains a nucleotide sequence encoding a functional endogenous mouse MHC II polypeptide. In various embodiments, the genetically modified rodent (e.g., mouse, rat, etc.) comprises in its germline a nucleotide sequence encoding a humanized MHC II complex, e.g., a humanized MHC II α and/or β polypeptide(s).

Disclosed herein is a rodent, e.g., a rat or a mouse, comprising in its genome, e.g., at an endogenous rodent MHC II locus, a nucleotide sequence encoding a human MHC II polypeptide. Disclosed herein is a rodent, e.g., a rat or a mouse, comprising in its genome, e.g., at an endogenous MHC II locus, a nucleotide sequence encoding a chimeric human/rodent MHC II polypeptide. Thus, also disclosed herein is a rodent that comprises in its genome, e.g., at an endogenous rodent MHC II locus, a nucleotide sequence(s) encoding a human or a chimeric human/rodent MHC II complex.

Disclosed herein is a chimeric human/rodent MHC II complex. In one example, the chimeric human/rodent MHC II complex comprises a chimeric human/rodent MHC II α polypeptide and a chimeric human/rodent MHC II β polypeptide. In one example, a human portion of the chimeric MHC II α polypeptide and/or a human portion of the chimeric MHC II β polypeptide comprises a peptide-binding domain of a human MHC II α polypeptide and/or human MHC II β polypeptide, respectively. In one example, a human portion of the chimeric MHC II α and/or β polypeptide comprises an extracellular domain of a human MHC II α and/or β polypeptide, respectively. In one example, a human portion of the chimeric MHC II α polypeptide comprises α1 domain of a human MHC II α polypeptide; in another embodiment, a human portion of the chimeric MHC II α polypeptide comprises α1 and α2 domains of a human MHC II α polypeptide. In an additional example, a human portion of the chimeric MHC II β polypeptide comprises β1 domain of a human MHC II β polypeptide; in another embodiment, a human portion of the chimeric MHC II β polypeptide comprises β1 and β2 domains of a human MHC II β polypeptide.

The human portion of the MHC II α and β polypeptides described herein may be encoded by any of -DQ, and -DR loci according to the claims. A list of commonly used HLA antigens and alleles is described in Shankarkumar et al. ((2004) The Human Leukocyte Antigen (HLA) System, Int. J. Hum. Genet. 4(2):91-103). Shankarkumar et al. also present a brief explanation of HLA nomenclature used in the art. Additional information regarding HLA nomenclature and various HLA alleles can be found in Holdsworth et al. (2009) The HLA dictionary 2008: a summary of HLA-A, -B, -C, -DRB1/3/4/5, and DQB1 alleles and their association with serologically defined HLA-A, -B, -C, -DR, and -DQ antigens, Tissue Antigens 73:95-170, and a recent update by Marsh et al. (2010) Nomenclature for factors of the HLA system, 2010, Tissue Antigens 75:291-455. Thus, the human or humanized MHC II polypeptide may be derived from any functional human HLA molecules described therein.

In one specific aspect, the human portions of the humanized MHC II complex described herein are derived from human, HLA-DR4 or HLA-DR2 according to the claims. Typically, HLA-DR α chains are monomorphic, e.g., the α chain of HLA-DR complex is encoded by HLA-DRA gene (e.g., HLA-DRα1^{∗}01 gene). On the other hand, the HLA-DR β chain is polymorphic. Thus, HLA-DR4 comprises an α chain encoded by HLA-DRA gene and a β chain encoded by HLA-DRB1 gene (e.g., HLA-DRβ1^{∗}04 gene). As described herein below, HLA-DR4 is known to be associated with incidence of a number of autoimmune diseases, e.g., rheumatoid arthritis, type I diabetes, multiple sclerosis, etc. HLA-DR2 comprises an α chain encoded by HLA-DRA gene and a β chain encoded by HLA-DRB1 gene (e.g., HLA-DRβ1^{∗}02 gene). HLA-DR2 is known to be associated with a number of diseases, e.g., Goodpasture syndrome, multiple sclerosis, etc. In one embodiment of the invention, the HLA-DRA allele is HLA-DRα^{∗}01 allele, e.g., HLA-DRα^{∗}01:01:01:01. In another embodiment, the HLA-DRB allele is HLA-DRB1^{∗}04, e.g., HLA-DRβ1^{∗}04:01:01. In another embodiment, the HLA-DRB allele is HLA- DRβ1^{∗}02, e.g., HLA- DRβ1^{∗}1501.

In another specific embodiment, the human portions of the humanized MHC II complex described herein are derived from human, HLA-DQ2 and HLA-DQ8 according to the claims. HLA-DQ2 comprises an α chain encoded by HLA-DQA gene (e.g., HLA-DQα1^{∗}05 gene). In one embodiment, HLA-DQα1^{∗}05 gene is HLA-DQα1^{∗}0501. HLA-DQ2 also comprises a β chain encoded by HLA-DQB gene (e.g., HLA-DQβ1^{∗}02 gene). HLA-DQ8 comprises an α chain encoded by HLA-DQA gene (e.g., HLA-DQα1^{∗}0301 gene). HLA-DQ8 also comprises a β chain encoded by HLA-DQB gene (e.g., HLA-DQβ1^{∗}0302 gene). HLA-DQ2.5 and HLA-DQ8 alleles are known to be associated with such diseases as Celiac disease and type I diabetes.

Although the present Examples describe these particular HLA sequences; any suitable HLA-DR2, HLA-DR4, HLA-DQ2 or HLA-DQ8 sequences are encompassed herein, e.g., polymorphic variants exhibited in human population, sequences with one or more conservative or non-conservative amino acid modifications, nucleic acid sequences differing from the sequences described herein due to the degeneracy of genetic code, etc.

The human portions of the humanized MHC II complex may be encoded by nucleotide sequences of HLA alleles known to be associated with common human diseases. Such HLA alleles include, but are not limited to, HLA-DRB1^{∗}0401, -DRB1^{∗}0301, - DQA1^{∗}0501, -DQB1^{∗}0201, -DRB1^{∗}1501, -DRB1^{∗}1502, -DQB1^{∗}0602, -DQA1^{∗}0102, - DQA1^{∗}0201, -DQB1^{∗}0202, -DQA1^{∗}0501, and combinations thereof. For a summary of HLA allele/disease associations, see Bakker et al. (2006) A high-resolution HLA and SNP haplotype map for disease association studies in the extended human MHC, Nature Genetics 38:1166-72 and Supplementary Information.

In the present invention, a rodent portion of the chimeric human/rodent MHC II complex comprises transmembrane and/or cytoplasmic domains of an endogenous rodent (e.g., mouse, rat, etc.) MHC II complex. Thus, a rodent portion of the chimeric human/rodent MHC II α polypeptide comprises transmembrane and cytoplasmic domains of an endogenous rodent MHC II α polypeptide. A rodent portion of the chimeric human/rodent MHC II β polypeptide comprises transmembrane and/or cytoplasmic domains of an endogenous rodent MHC II β polypeptide. In one aspect, the animal is a mouse, and rodent portions of the chimeric α and β polypeptides are derived from a mouse H-2E protein. Thus, rodent portions of the chimeric α and β polypeptides may comprise transmembrane and cytoplasmic domains derived from a mouse H-2E protein. In another aspect, the animal is a mouse, and rodent portions of the chimeric α and β polypeptides are derived from a mouse H-2A protein. Thus, rodent portions of the chimeric α and β polypeptides may comprise transmembrane and cytoplasmic domains derived from a mouse H-2A protein. Although specific H-2E and H-2A sequences are contemplated in the Examples, any suitable sequences, e.g., polymorphic variants, conservative/non-conservative amino acid substitutions, etc., are encompassed herein.

In the present invention, the sequence(s) encoding a chimeric human/rodent MHC II complex are located at an endogenous rodent MHC II locus (e.g., mouse H-2A and/or H-2E locus). In one embodiment, this results in a replacement of an endogenous MHC II gene(s) or a portion thereof with a nucleotide sequence(s) encoding a humanized MHC II protein, e.g., a chimeric gene encoding a chimeric human/rodent MHC II protein described herein. Since the nucleotide sequences encoding MHC II α and β polypeptides are located in proximity to one another on the chromosome, a replacement can be designed to target the two genes either independently or together; both of these possibilities are encompassed herein. In one embodiment, the replacement comprises a replacement of an endogenous nucleotide sequence encoding an MHC II α and β polypeptides with a nucleotide sequence encoding a chimeric human/rodent MHC α polypeptide and a chimeric human/rodent MHC β polypeptide. In one aspect, the replacement comprises replacing nucleotide sequences representing one or more (e.g., two) endogenous MHC II genes. Thus, the rodent contains a chimeric human/rodent nucleotide sequence at an endogenous MHC II locus, and expresses a chimeric human/rodent MHC II protein from the endogenous rodent locus.

Thus, disclosed herein is a rodent comprising at an endogenous MHC II gene locus a first nucleotide sequence encoding a chimeric human/rodent MHC II α polypeptide and a second nucleotide sequence encoding a chimeric human/rodent MHC II β polypeptide, wherein a human portion of the chimeric human/rodent MHC II α polypeptide comprises a human MHC II α extracellular domain and a human portion of the chimeric human/rodent MHC II β polypeptide comprises a human MHC II β extracellular domain, and wherein the chimeric human/rodent MHC II α and MHC II β polypeptides form a functional MHC II complex on a surface of a cell.

A chimeric human/rodent polypeptide may be such that it comprises a human or a rodent leader (signal) sequence. In one embodiment, the chimeric MHC II α polypeptide comprises a rodent leader sequence of an endogenous MHC II α polypeptide. In one embodiment, the chimeric MHC II β polypeptide comprises a rodent leader sequence of an endogenous MHC II β polypeptide. In an alternative embodiment, the chimeric MHC II α and/or MHC II β polypeptide comprises a rodent leader sequence of MHC II α and/or MHC II β polypeptide, respectively, from another rodent, e.g. another mouse strain. Thus, the nucleotide sequence encoding the chimeric MHC II α and/or MHC II β polypeptide may be operably linked to a nucleotide sequence encoding a rodent MHC II α and/or MHC II β leader sequence, respectively. In yet another embodiment, the chimeric MHC II α and/or MHC II β polypeptide comprises a human leader sequence of human MHC II α and/or human MHC II β polypeptide, respectively. In one embodiment, the human leader sequence of the human MHC II α is a leader sequence of human HLA-DRA and the human leader sequence of the human MHC II β polypeptide is a leader sequence of human HLA-DRβ 1 *04. In another embodiment, the human leader sequence of the human MHC II α is a leader sequence of human HLA-DRA and the human leader sequence of the human MHC II β polypeptide is a leader sequence of human HLA-DRβ 1 *02. In another embodiment, the human leader sequence of the human MHC II α is a leader sequence of human HLA-DQα1^{∗}05 and the human leader sequence of the human MHC II β polypeptide is a leader sequence of human HLA-DQβ1^{∗}02. In yet another embodiment, the human leader sequence of the human MHC II α is a leader sequence of human HLA-DQα1^{∗}0301 and the human leader sequence of the human MHC II β polypeptide is a leader sequence of human HLA-DQβ1^{∗}0302.

A chimeric human/rodent MHC II α and/or MHC II β polypeptide may comprise in its human portion a complete or substantially complete extracellular domain of a human MHC II α and/or human MHC II β polypeptide, respectively. Thus, a human portion may comprise at least 80%, preferably at least 85%, more preferably at least 90%, e.g., 95% or more of the amino acids encoding an extracellular domain of a human MHC II α and/or human MHC II β polypeptide. In one example, substantially complete extracellular domain of the human MHC II α and/or human MHC II β polypeptide lacks a human leader sequence. In another example, the chimeric human/rodent MHC II α and/or the chimeric human/rodent MHC II β polypeptide comprises a human leader sequence.

Moreover, the chimeric MHC II α and/or MHC II β polypeptide may be operably linked to (e.g., may be expressed under the control of) endogenous rodent promoter and regulatory elements, e.g., mouse MHC II α and/or MHC II β regulatory elements, respectively. Such arrangement will facilitate proper expression of the chimeric MHC II polypeptides in the non-human animal, e.g., during immune response in the rodent.

The genetically modified rodent may be selected from a group consisting of a mouse or rat. For the rodents where suitable genetically modifiable ES cells are not readily available, other methods are employed to make a rodent comprising the genetic modification. Such methods include, e.g., modifying a non-ES cell genome (e.g., a fibroblast or an induced pluripotent cell) and employing nuclear transfer to transfer the modified genome to a suitable cell, e.g., an oocyte, and gestating the modified cell (e.g., the modified oocyte) in a rodent under suitable conditions to form an embryo.

In one aspect, the rodent is of the superfamily Dipodoidea or Muroidea. The genetically modified animal is a rodent. In one embodiment, the rodent is selected from a mouse, a rat, and a hamster. In one embodiment, the rodent is selected from the superfamily Muroidea. In one embodiment, the genetically modified animal is from a family selected from Calomyscidae *(e.g*., mouse-like hamsters), Cricetidae (e.g., hamster, New World rats and mice, voles), Muridae (true mice and rats, gerbils, spiny mice, crested rats), Nesomyidae (climbing mice, rock mice, with-tailed rats, Malagasy rats and mice), Platacanthomyidae (e.g., spiny dormice), and Spalacidae (e.g., mole rates, bamboo rats, and zokors). In a specific embodiment, the genetically modified rodent is selected from a true mouse or rat (family Muridae), a gerbil, a spiny mouse, and a crested rat. In one embodiment, the genetically modified mouse is from a member of the family Muridae. In the present invention, the animal is a rodent. In a specific embodiment, the rodent is selected from a mouse and a rat. In one embodiment, the rodent is a mouse.

In a specific embodiment, the rodent is a mouse of a C57BL strain selected from C57BL/A, C57BL/An, C57BL/GrFa, C57BL/KaLwN, C57BL/6, C57BL/6J, C57BL/6ByJ, C57BL/6NJ, C57BL/10, C57BL/10ScSn, C57BL/10Cr, and C57BL/Ola. In another embodiment, the mouse is a 129 strain selected from the group consisting of a strain that is 129P1, 129P2, 129P3, 129X1, 129S1 (*e.g*., 129S1/SV, 129S1/SvIm), 129S2, 129S4, 129S5, 129S9/SvEvH, 129S6 (129/SvEvTac), 129S7, 129S8, 129T1, 129T2 (see, *e.g*., Festing et al. (1999) Revised nomenclature for strain 129 mice, Mammalian Genome 10:836, *see also,* Auerbach et al (2000) Establishment and Chimera Analysis of 129/SvEv- and C57BL/6-Derived Mouse Embryonic Stem Cell Lines). In a specific embodiment, the genetically modified mouse is a mix of an aforementioned 129 strain and an aforementioned C57BL/6 strain. In another specific embodiment, the mouse is a mix of aforementioned 129 strains, or a mix of aforementioned BL/6 strains. In a specific embodiment, the 129 strain of the mix is a 129S6 (129/SvEvTac) strain. In another embodiment, the mouse is a BALB strain, e.g., BALB/c strain. In yet another embodiment, the mouse is a mix of a BALB strain and another aforementioned strain.

In one embodiment, the rodent is a rat. In one embodiment, the rat is selected from a Wistar rat, an LEA strain, a Sprague Dawley strain, a Fischer strain, F344, F6, and Dark Agouti. In one embodiment, the rat strain is a mix of two or more strains selected from the group consisting of Wistar, LEA, Sprague Dawley, Fischer, F344, F6, and Dark Agouti.

Thus, in one embodiment, the invention relates to a genetically modified mouse that comprises in its genome a nucleotide sequence encoding a chimeric human/mouse MHC II complex, e.g., chimeric human/mouse MHC II α and β polypeptides. A human portion of the chimeric human/mouse MHC II α polypeptide comprises a human MHC II α peptide binding or extracellular domain and a human portion of the chimeric human/mouse MHC II β polypeptide comprises a human MHC II β peptide binding or extracellular domain. In some embodiments, the mouse does not express a peptide binding or an extracellular domain of endogenous mouse α and/or β polypeptide from an endogenous mouse locus (e.g., H-2A and/or H-2E locus). In some embodiments, the mouse does not express functional peptide binding or extracellular domains of endogenous mouse MHC II polypeptides from endogenous mouse MHC II locus. In some embodiments, the mouse comprises a genome that lacks a gene that encodes a functional MHC class II molecule comprising an H-2Ab1, H-2Aa, H-2Eb1, H-2Eb2, H-2Ea, and a combination thereof. The peptide-binding domain of the human MHC II α polypeptide may comprise α1 domain and the peptide-binding domain of the human MHC II β polypeptide may comprise a β1 domain; thus, the peptide-binding domain of the chimeric MHC II complex may comprise human α1 and β1 domains. The extracellular domain of the human MHC II α polypeptide comprises α1 and α2 domains and the extracellular domain of the human MHC II β polypeptide comprises β1 and β2 domains; thus, the extracellular domain of the chimeric MHC II complex comprises human α1, α2, β1 and β2 domains. In one embodiment, the mouse portion of the chimeric MHC II complex comprises transmembrane and cytosolic domains of mouse MHC II, e.g. mouse H-2E or H-2A (e.g., transmembrane and cytosolic domains of mouse H-2E α and β chains or mouse H-2A α and β chains).

Therefore, in one embodiment, a genetically modified mouse is provided, wherein the mouse comprises at an endogenous mouse MHC II locus a first nucleotide sequence encoding a chimeric human/mouse MHC II α polypeptide and a second nucleotide sequence encoding a chimeric human/mouse MHC II β polypeptide, wherein a human portion of the chimeric MHC II α polypeptide comprises an extracellular domain derived from an α polypeptide of a human HLA-DR4 protein and a human portion of the chimeric MHC II β polypeptide comprises an extracellular domain derived from a β polypeptide of a human HLA-DR4 protein, wherein a mouse portion of the chimeric MHC II α polypeptide comprises transmembrane and cytoplasmic domains of a mouse H-2E α chain and a mouse portion of the chimeric MHC II β polypeptide comprises transmembrane and cytoplasmic domains of a mouse H-2E β chain, and wherein the mouse expresses a functional chimeric HLA-DR4/H-2E MHC II complex. In one embodiment the chimeric HLA-DR4/H-2E MHC II complex comprises an MHC II α chain that includes extracellular domains α1, and α2 domains) derived from HLA-DR4 protein (HLA-DRA α1, and α2 domains) and transmembrane and cytoplasmic domains from a mouse H-2E α chain, as well as an MHC II β chain that includes extracellular domains β1 and β2 domains) derived from HLA-DR4 (HLA-DRβ1^{∗}04 β1 and β2 domains) and transmembrane and cytoplasmic domains from mouse H-2E β chain.

In another embodiment, a genetically modified mouse is provided, wherein the mouse comprises at an endogenous mouse MHC II locus a first nucleotide sequence encoding a chimeric human/mouse MHC II α polypeptide and a second nucleotide sequence encoding a chimeric human/mouse MHC II β polypeptide, wherein a human portion of the chimeric MHC II α polypeptide comprises an extracellular domain derived from an α polypeptide of a human HLA-DR2 protein and a human portion of the chimeric MHC II β polypeptide comprises an extracellular domain derived from a β polypeptide of a human HLA-DR2 protein, wherein a mouse portion of the chimeric MHC II α polypeptide comprises transmembrane and cytoplasmic domains of a mouse H-2E α chain and a mouse portion of the chimeric MHC II β polypeptide comprises transmembrane and cytoplasmic domains of a mouse H-2E β chain, and wherein the mouse expresses a functional chimeric HLA-DR2/H-2E MHC II complex. In one embodiment the chimeric HLA-DR2/H-2E MHC II complex comprises an MHC II α chain that includes extracellular domains (e.g., α1, and α2 domains) derived from HLA-DR2 protein (HLA-DRA α1, and α2 domains) and transmembrane and cytoplasmic domains from a mouse H-2E α chain, as well as an MHC II β chain that includes extracellular domains (e.g., β1 and β2 domains) derived from HLA-DR2 (HLA-DRβ1^{∗}02 β1 and β2 domains) and transmembrane and cytoplasmic domains from mouse H-2E β chain.

In another embodiment, a genetically modified mouse is provided, wherein the mouse comprises at an endogenous mouse MHC II locus a first nucleotide sequence encoding a chimeric human/mouse MHC II α polypeptide and a second nucleotide sequence encoding a chimeric human/mouse MHC II β polypeptide, wherein a human portion of the chimeric MHC II α polypeptide comprises an extracellular domain derived from an α polypeptide of a human HLA-DQ2 protein and a human portion of the chimeric MHC II β polypeptide comprises an extracellular domain derived from a β polypeptide of a human HLA-DQ2 protein, wherein a mouse portion of the chimeric MHC II α polypeptide comprises transmembrane and cytoplasmic domains of a mouse H-2A α chain and a mouse portion of the chimeric MHC II β polypeptide comprises transmembrane and cytoplasmic domains of a mouse H-2A β chain, and wherein the mouse expresses a functional chimeric HLA-DQ2/H-2A MHC II complex. In one embodiment the chimeric HLA-DQ2/H-2A MHC II complex comprises an MHC II α chain that includes extracellular domains (e.g., α1, and α2 domains) derived from HLA-DQ2 protein (HLA-DQα1^{∗}05 α1, and α2 domains) and transmembrane and cytoplasmic domains from a mouse H-2A α chain, as well as an MHC II β chain that includes extracellular domains (e.g., β1 and β2 domains) derived from HLA-DQ2 (HLA-DQβ1^{∗}02 β1 and β2 domains) and transmembrane and cytoplasmic domains from mouse H-2A β chain.

In yet another embodiment, a genetically modified mouse is provided, wherein the mouse comprises at an endogenous mouse MHC II locus a first nucleotide sequence encoding a chimeric human/mouse MHC II α polypeptide and a second nucleotide sequence encoding a chimeric human/mouse MHC II β polypeptide, wherein a human portion of the chimeric MHC II α polypeptide comprises an extracellular domain derived from an α polypeptide of a human HLA-DQ8 protein and a human portion of the chimeric MHC II β polypeptide comprises an extracellular domain derived from a β polypeptide of a human HLA-DQ8 protein, wherein a mouse portion of the chimeric MHC II α polypeptide comprises transmembrane and cytoplasmic domains of a mouse H-2A α chain and a mouse portion of the chimeric MHC II β polypeptide comprises transmembrane and cytoplasmic domains of a mouse H-2A β chain, and wherein the mouse expresses a functional chimeric HLA-DQ8/H-2A MHC II complex. In one embodiment the chimeric HLA-DQ8/H-2A MHC II complex comprises an MHC II α chain that includes extracellular domains (e.g., α1, and α2 domains) derived from HLA-DQ8 protein (HLA-DQα1^{∗}0301 α1, and α2 domains) and transmembrane and cytoplasmic domains from a mouse H-2A α chain, as well as an MHC II β chain that includes extracellular domains (e.g., β1 and β2 domains) derived from HLA-DQ8 (HLA-DQβ1^{∗}0302 β1 and β2 domains) and transmembrane and cytoplasmic domains from mouse H-2A β chain.

In one aspect, the mouse does not express functional endogenous H-2A and H-2E polypeptides from their endogenous mouse loci (e.g., the mouse does not express H-2Ab1, H-2Aa, H-2Eb1, H-2Eb2, and H-2Ea polypeptides). In various embodiments, expression of the first and second nucleotide sequences is under the control of respective endogenous mouse promoters and regulatory elements (e.g., the first and second nucleotide sequences are operably linked to endogenous promoters and regulatory elements). In various embodiments of the invention, the first and the second nucleotide sequences are located on the same chromosome. In some aspects, the mouse comprises two copies of the chimeric MHC II locus containing the first and the second nucleotide sequences, while in other aspects, the mouse comprises one copy of the MHC II locus containing the first and the second nucleotide sequences. Thus, the mouse may be homozygous or heterozygous for the chimeric MHC II locus containing the first and the second nucleotide sequences. In various embodiments, the first and the second nucleotide sequences are comprised in the germline of the mouse.

In some embodiments described herein, a mouse is provided that comprises a chimeric MHC II locus at an endogenous mouse MHC II locus, e.g., via replacement of endogenous mouse H-2A and H-2E genes. In some aspects, the chimeric locus comprises a nucleotide sequence that encodes an extracellular domain of a human HLA-DRA and transmembrane and cytoplasmic domains of a mouse H-2E α chain, as well as an extracellular domain of a human HLA-DRβ1^{∗}04 or HLA- DRβ1^{∗}02 and transmembrane and cytoplasmic domains of a mouse H-2E β chain. In other aspects, the chimeric locus comprises a nucleotide sequence that encodes an extracellular domain of a human HLA-DQα1^{∗}05 or HLA-DQα1^{∗}0301 and transmembrane and cytoplasmic domains of a mouse H-2A α chain, as well as an extracellular domain of a human HLA-DQβ1^{∗}02 or HLA-DQβ1^{∗}0302 and transmembrane and cytoplasmic domains of a mouse H-2Aβ chain. The various domains of the chimeric locus are linked in such a fashion that the locus expresses a functional chimeric human/mouse MHC II complex.

In the present invention, a rodent (e.g., a mouse or rat) that expresses a functional chimeric MHC II protein from a chimeric MHC II locus as described herein displays the chimeric protein on a cell surface. In one embodiment, the rodent expresses the chimeric MHC II protein on a cell surface in a cellular distribution that is the same as observed in a human. In one example, the cell displays a peptide fragment (antigen fragment) bound to an extracellular portion (e.g., human HLA-DR4, -DR2, -DQ2, or -DQ8 extracellular portion) of the chimeric MHC II protein.

In various embodiments, a cell displaying the chimeric MHC II protein (e.g., HLA-DR4/H-2E, HLA-DR2/H-2E, HLA-DQ2/H-2A, or HLA-DQ8/H-2A protein) is an antigen-presenting cell (APC) e.g., a macrophage, a dendritic cell, or a B cell. In some embodiments, the peptide fragment presented by the chimeric protein is derived from a tumor. In other embodiments, the peptide fragment presented by the chimeric MHC II protein is derived from a pathogen, e.g., a bacterium, a virus, or a parasite.

The chimeric MHC II protein described herein may interact with other proteins on the surface of the same cell or a second cell. In some embodiments, the chimeric MHC II protein interacts with endogenous rodent proteins on the surface of said cell. The chimeric MHC II protein may also interact with human or humanized proteins on the surface of the same cell or a second cell. In some embodiments, the second cell is a T cell, and the chimeric MHC II protein interacts with T cell receptor (TCR) and its co-receptor CD4. In some embodiments, the T cell is an endogenous mouse T cell. In other embodiments, the T cell is a human T cell. In some embodiments, the TCR is a human or humanized TCR. In additional embodiments, the CD4 is a human or humanized CD4. In other embodiment, either one or both of TCR and CD4 are non-human, e.g., mouse or rat.

A genetically modified rodent as described herein is disclosed that does not develop tumors at a higher rate than a wild-type animal that lacks a chimeric MHC II gene. In some examples the animal does not develop hematological malignancies, e.g., various T and B cell lymphomas, leukemias, composite lymphomas (e.g., Hodgkin's lymphoma), at a higher rate than the wild-type animal.

In addition to a genetically engineered rodent, a rodent embryo e.g., a mouse or a rat embryo) is also disclosed wherein the embryo comprises a donor ES cell that is derived from a rodent (e.g., a mouse or a rat) as described herein. In one example, the embryo comprises an ES donor cell that comprises the chimeric MHC II gene, and host embryo cells.

Also disclosed herein is a tissue, wherein the tissue is derived from a rodent (e.g., a mouse or a rat) as described herein, and expresses the chimeric MHC II protein (e.g., HLA-DR4/H-2E, HLA-DR2/H-2E, HLA-DQ2/H-2A, or HLA-DQ8/H-2A protein).

In addition, a rodent cell isolated from a rodent as described herein is disclosed. In one embodiment, the cell is an ES cell. In one embodiment, the cell is an antigen-presenting cell, e.g., dendritic cell, macrophage, B cell. In one embodiment, the cell is an immune cell. In one embodiment, the immune cell is a lymphocyte.

Also disclosed is a rodent cell comprising a chromosome or fragment thereof of a rodent as described herein. In one embodiment, the rodent cell comprises a nucleus of a rodent as described herein. In one embodiment, the rodent cell comprises the chromosome or fragment thereof as the result of a nuclear transfer.

Disclosed herein is a rodent induced pluripotent cell comprising gene encoding a chimeric MHC II protein (e.g., HLA-DR4/H-2E, HLA-DR2/H-2E, HLA-DQ2/H-2A, or HLA-DQ8/H-2A protein) as described herein is provided. In one embodiment, the induced pluripotent cell is derived from a non rodent as described herein.

Disclosed herein is a hybridoma or quadroma, derived from a cell of a rodent as described herein. In one embodiment, the rodent is a mouse or rat.

Disclosed herein is an *in vitro* preparation that comprises a first cell that bears a chimeric human/rodent MHC II surface protein that comprises a bound peptide to form a chimeric human/rodent MHC II/peptide complex, and a second cell that binds the chimeric human/rodent MHC II/peptide complex. In one embodiment, the second cell comprises a human or humanized T-cell receptor, and in one embodiment further comprises a human or humanized CD4. In one embodiment, the second cell is a rodent (e.g., mouse or rat) cell comprising a human or humanized T-cell receptor and a human or humanized CD4 protein. In one embodiment, the second cell is a human cell.

Also provided is a method for making a genetically engineered rodent (e.g., a genetically engineered mouse or rat) described herein. The method for making a genetically engineered rodent results in the animal whose genome comprises a nucleotide sequence encoding a chimeric MHC II protein (e.g., chimeric MHC II α and β polypeptides). In one embodiment, the method results in a genetically engineered mouse, whose genome comprises at an endogenous MHC II locus a nucleotide sequence encoding a chimeric human/mouse MHC II protein, wherein a human portion of the chimeric MHC II protein comprises an extracellular domain of a human HLA-DR4 or HLA-DR2 and a mouse portion comprises transmembrane and cytoplasmic domains of a mouse H-2E. In another embodiment, the method results in a genetically engineered mouse, whose genome comprises at an endogenous MHC II locus a nucleotide sequence encoding a chimeric human/mouse MHC II protein, wherein a human portion of the chimeric MHC II protein comprises an extracellular domain of a human HLA-DQ2 or HLA-DQ8 and a mouse portion comprises transmembrane and cytoplasmic domains of a mouse H-2A. In some embodiments, the method utilizes a targeting construct made using VELOCIGENE^{®} technology, introducing the construct into ES cells, and introducing targeted ES cell clones into a mouse embryo using VELOCIMOUSE^{®} technology, as described in the Examples. In one embodiment, the ES cells are a mix of 129 and C57BL/6 mouse strains; in one embodiment, the ES cells are a mix of BALB/c and 129 mouse strains.

A nucleotide construct used for generating genetically engineered rodents described herein is also disclosed. In one example, the nucleotide construct comprises: 5' and 3' non-human homology arms, a DNA fragment comprising human HLA-DR α and β chain sequences, and a selection cassette flanked by recombination sites. In one example, the human HLA-DR α and β chain sequences are genomic sequences that comprise introns and exons of human HLA-DR α and β chain genes. In one embodiment, the rodent homology arms are homologous to rodent MHC II genomic sequence.

In one example, the human HLA-DR α chain sequence comprises an α1 and α2 domain coding sequence. In a specific embodiment, it comprises, from 5' to 3': α1 exon (exon 2), α1/α2 intron (intron 2), and α2 exon (exon 3). In one embodiment, the human HLA-DR β chain sequence comprises a β1 and β2 domain coding sequence. In a specific embodiment, it comprises, from 5' to 3': β1 exon (exon 2), β1/β2 intron (intron 2), and β2 exon (exon 3).

Similarly, also disclosed herein are nucleotide constructs for generating genetically engineered rodents comprising human HLA-DQ α and β chain sequences (e.g., human HLA-DQ α1 and α2 domain coding sequences and human HLA-DQ β1 and β2 domain coding sequences). Specific embodiments of such constructs are presented in FIG. 10.

A selection cassette is a nucleotide sequence inserted into a targeting construct to facilitate selection of cells (e.g., ES cells) that have integrated the construct of interest. A number of suitable selection cassettes are known in the art. Commonly, a selection cassette enables positive selection in the presence of a particular antibiotic (e.g., Neo, Hyg, Pur, CM, SPEC, etc.). In addition, a selection cassette may be flanked by recombination sites, which allow deletion of the selection cassette upon treatment with recombinase enzymes. Commonly used recombination sites are *lox*P and *Frt,* recognized by Cre and Flp enzymes, respectively, but others are known in the art. A selection cassette may be located anywhere in the construct outside the coding region. In one example, the selection cassette is located in the β chain intron, e.g., β2/transmembrane domain intron (intron 3).

In one embodiment, 5' and 3' homology arms comprise genomic sequence at 5' and 3' locations of endogenous non-human MHC II locus. In one embodiment, the 5' homology arm comprises genomic sequence upstream of mouse H-2Ab2 gene and the 3' homology arm comprises genomic sequence downstream of mouse H-2Ea gene. In this embodiment, the construct allows replacement of both mouse H-2E and H-2A genes.

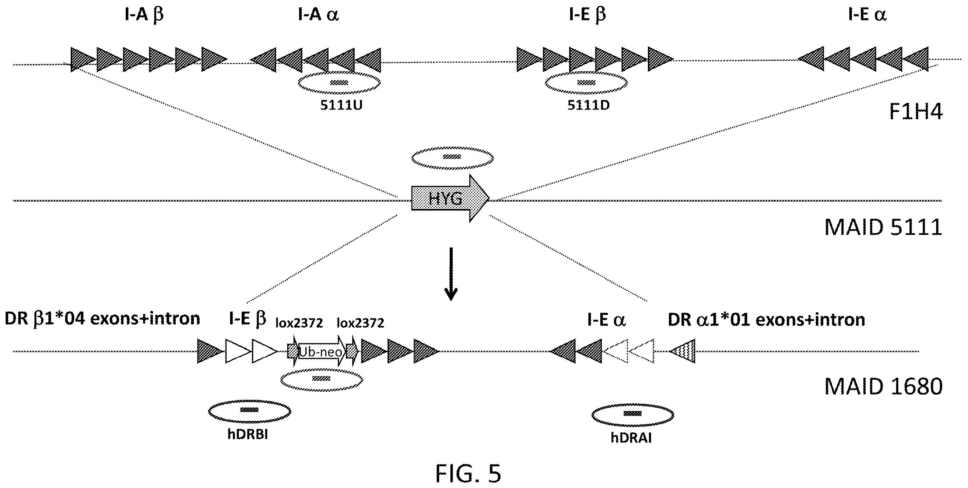

Thus, in one example, a nucleotide construct is disclosed comprising, from 5' to 3': a 5' homology arm containing mouse genomic sequence upstream of mouse H-2Ab2 gene, a first nucleotide sequence comprising a sequence encoding a chimeric human/mouse MHC II β chain, a second nucleotide sequence comprising a sequence encoding a chimeric human/mouse MHC II α chain, and a 3' homology arm containing mouse genomic sequence downstream of mouse H-2Ea gene. In a specific example, the first nucleotide sequence comprising a sequence encoding a chimeric human/mouse MHC II β chain comprises human β1 exon, β1/β2 intron, β2 exon, an a selection cassette flanked by recombination sites inserted in the intronic region between the human β2 exon sequence and the sequence of a mouse transmembrane domain exon. In a specific example, the second nucleotide sequence comprising a sequence encoding a chimeric human/mouse MHC II α chain comprises human α1 exon, α1/α2 intron, and human α2 exon. Exemplary constructs of the invention are depicted in FIGs. 5 and 10.

Upon completion of gene targeting, ES cells or genetically modified rodents are screened to confirm successful incorporation of exogenous nucleotide sequence of interest or expression of exogenous polypeptide. Numerous techniques are known to those skilled in the art, and include (but are not limited to) Southern blotting, long PCR, quantitative PCT (e.g., real-time PCR using TAQMAN^{®}), fluorescence *in situ* hybridization, Northern blotting, flow cytometry, Western analysis, immunocytochemistry, immunohistochemistry, etc. In one example, rodents (e.g., mice) bearing the genetic modification of interest can be identified by screening for loss of mouse allele and/or gain of human allele using a modification of allele assay described in Valenzuela et al. (2003) High-throughput engineering of the mouse genome coupled with high-resolution expression analysis, Nature Biotech. 21(6):652-659. Other assays that identify a specific nucleotide or amino acid sequence in the genetically modified animals are known to those skilled in the art.

The disclosure also provides a method of modifying an MHC II locus of a mouse to express a chimeric human/rodent MHC II complex described herein. In one embodiment, the invention provides a method of modifying an MHC II locus of a mouse to express a chimeric human/mouse MHC II complex comprising replacing at the endogenous mouse MHC II locus a nucleotide sequence encoding a mouse MHC II complex with a example, nucleotide sequence encoding a chimeric human/mouse MHC II complex. In a specific aspect, the nucleotide sequence encoding the chimeric human/mouse MHC II complex comprises a first nucleotide sequences encoding an extracellular domain of a human MHC II α chain (HLA-DR2, HLA-DR4, HLA-DQ2, HLA-DQ2.5, HLA-DQ8, or HLA-DQ8.1 α chain) and transmembrane and cytoplasmic domains of a mouse MHC II α chain (e.g., H-2E or H-2A α chain) and a second nucleotide sequence encoding an extracellular domain of a human MHC II β chain (HLA-DR2, HLA-DR4, HLA-DQ2, HLA-DQ2.5, HLA-DQ8, or HLA-DQ8.1 β chain) and transmembrane and cytoplasmic domains of a mouse MHC II β chain (e.g., H-2E or H-2A β chain, e.g., H-2Eb1 or H-2Ab1 chain, respectively). In some examples, the modified mouse MHC II locus expresses a chimeric HLA-DR4/H-2E protein. In other examples, the modified mouse MHC II locus expresses a chimeric HLA-DR2/H-2E protein, chimeric HLA-DQ2/H-2A protein, or chimeric HLA-DQ8/H-2A protein.

A method for making a chimeric human HLA class II/rodent MHC class II molecule is disclosed, comprising expressing in a single cell a chimeric human/rodent MHC II protein (e.g., chimeric HLA-DR4/H-2E, HLA-DR2/H-2E, HLA-DQ2/H-2A, or HLA-DQ8/H-2A protein) from a nucleotide construct as described herein. In one embodiment, the nucleotide construct is a viral vector; in a specific embodiment, the viral vector is a lentiviral vector. In one embodiment, the cell is selected from a CHO, COS, 293, HeLa, and a retinal cell expressing a viral nucleic acid sequence (e.g., a PERC.6^{™} cell).

Disclosed herein is a cell that expresses a chimeric HLA-DR4/H-2E, HLA-DR2/H-2E, HLA-DQ2/H-2A, or HLA-DQ8/H-2A protein. In one example, the cell comprises an expression vector comprising a chimeric MHC class II sequence as described herein. In one example, the cell is selected from CHO, COS, 293, HeLa, and a retinal cell expressing a viral nucleic acid sequence (*e.g*., a PERC.6^{™} cell).

A chimeric MHC class II molecule made by a rodent as described herein is also disclosed, wherein the chimeric MHC class II molecule comprises α1, α2, β1, and β2 domains from a human MHC II protein, e.g., HLA-DR4, HLA-DR2, HLA-DQ2, or HLA-DQ8, and transmembrane and cytoplasmic domains from a non-human MHC II protein, e.g., mouse H-2E or H-2A protein. The chimeric MHC II complex comprising an extracellular domain of HLA-DR4 or HLA-DR2 described herein maybe detected by anti-HLA-DR antibodies. The chimeric MHC II complex comprising an extracellular domain of HLA-DQ2 or HLA-DQ8 described herein maybe detected by anti-HLA-DQ antibodies. Thus, a cell displaying chimeric human/non-human MHC II polypeptide may be detected and/or selected using anti-HLA-DR or anti-HLA-DQ antibody.

Although the Examples that follow describe a genetically engineered animal whose genome comprises a replacement of a nucleotide sequence encoding mouse H-2A and H-2E proteins with a nucleotide sequence encoding a chimeric human/mouse HLA-DR4/H-2E, HLA-DR2/H-2E, HLA-DQ2/H-2A, or HLA-DQ8/H-2A protein, C. In addition, introduction of multiple humanized MHC II molecules according to the claims (e.g., chimeric HLA-DR/H-2E and HLA-DQ/H-2A according to the claims) is also provided.

The replacement at multiple MHC II loci is provided. Thus, provided herein is a a rodent, e.g., a mouse, that comprises at an endogenous MHC II locus two or more, e.g., two, three, or four, nucleotide sequence(s) encoding a chimeric human/rodent (e.g., human/mouse) MHC II complex(es). In one instance, each of the two mouse sister chromosomes 17 contains an MHC II locus that comprises H-2A and H-2E genes; thus, in one aspect, each sister chromosome may encode up to two chimeric human/mouse complexes at their endogenous genomic positions. Therefore, in one embodiment, a genetically modified rodent, e.g., a mouse, may comprise up to four different nucleotide sequences encoding up to four chimeric human/rodent, e.g., human/mouse, MHC II complexes at their endogenous MHC loci.

In one embodiment, provided herein is a mouse comprising at an endogenous MHC II locus two or more, e.g., two, three, or four, nucleotide sequence(s) encoding a chimeric human/mouse MHC II complex(es) wherein a human portion of the MHC II complex comprises an extracellular domain of a human MHC II complex (i.e., extracellular domains of human MHC II α and β polypeptides) and wherein a mouse portion of the chimeric MHC II complex comprises a transmembrane and cytoplasmic domain of a mouse MHC II complex (i.e., transmembrane and cytoplasmic domains of a mouse MHC II α and β polypeptides, e.g., H-2A and H-2E transmembrane and extracellular domains), and wherein the mouse expresses one or more, e.g., one, two, three, or four, chimeric human/mouse MHC II complex(es). In one embodiment, the mouse MHC II is selected from H-2E and H-2A. In one embodiment, the human MHC II is selected from HLA-DR2, HLA-DR4, HLA-DQ2, HLA-DQ8.

Furthermore, disclosed herein is a method for generating a rodent, e.g., a mouse, comprising replacements at multiple endogenous MHC loci, e.g., a rodent, e.g., a mouse, comprising one or more, e.g., one, two, three, or four, nucleotide sequence(s) encoding a chimeric human/rodent, e.g., human/mouse, MHC II complex(es). Due to close linkage of the various MHC II loci on mouse chromosome 17, in some examples, the methods comprise successive replacements at the locus. In one example, the method comprises replacing a nucleotide sequence encoding a first mouse MHC II complex with a nucleotide sequence encoding a first chimeric human/mouse MHC II complex in an ES cell, generating a mouse expressing the first chimeric MHC II complex, generating an ES cell from said mouse, replacing in said ES cell a nucleotide sequence encoding a second mouse MHC II complex with a nucleotide sequence encoding a second chimeric human/mouse MHC II complex, and generating a mouse expressing two chimeric human/mouse MHC II complexes. Alternatively, the method may comprise replacing a nucleotide sequence encoding a first mouse MHC II complex with a nucleotide sequence encoding a first chimeric human/mouse MHC II complex in an ES cell, followed by replacing in same ES cell a nucleotide sequence encoding a second mouse MHC II complex with a nucleotide sequence encoding a second chimeric human/mouse MHC II complex, and generating a mouse expressing two chimeric human/mouse MHC II complexes. A mouse comprising a nucleotide sequence encoding a third or fourth chimeric human/mouse MHC II complex can be generated by, e.g., breeding. One skilled in the art would understand that a mouse comprising two or more chimeric MHC II complexes may also be generated by breeding rather than successive replacement; this animal may be heterozygous for all of the chimeric MHC II sequences (e.g., a mouse comprising a different chimeric gene on each of its sister chromosomes will be heterozygous for the two MHC genes, etc.). Alternatively to successive replacements at the locus, a mouse comprising more than one, e.g., two, nucleotide sequences encoding chimeric human/mouse MHC II complexes may be generated by replacing a nucleotide sequence encoding more than one, e.g., two, MHC II complexes with an exogenous nucleotide sequence encoding more than one, e.g., two, chimeric human/mouse MHC II complexes (e.g., nucleotide sequence encoding both mouse H-2A and H-2E genes may be replaced by a nucleotide sequence encoding chimeric HLA-DR/H-2E and HLA-DQ/H-2A complexes).

### Use of Genetically Modified Animals

In various embodiments, the genetically modified rodents described herein make APCs with humanized MHC II on the cell surface and, as a result, present peptides derived from cytosolic proteins as epitopes for T cells in a human-like manner, because substantially all of the components of the complex are human or humanized. The genetically modified rodents of the invention can be used to study the function of a human immune system in the humanized animal; for identification of antigens and antigen epitopes that elicit immune response (e.g., T cell epitopes, e.g., unique human cancer epitopes), e.g., for use in vaccine development; for evaluation of vaccine candidates and other vaccine strategies; for studying human autoimmunity; for studying human infectious diseases; and otherwise for devising better therapeutic strategies based on human MHC expression.

MHC II complex binds peptides derived from extracellular proteins, e.g., extracellular bacterium, neighboring cells, or polypeptides bound by B cell receptors and internalized into a B cell. Once extracellular proteins enter endocytic pathway, they are degraded into peptides, and peptides are bound and presented by MHC II. Once a peptide presented by MHC II is recognized by CD4+ T cells, T cells are activated, proliferate, differentiate to various T helper subtypes (e.g., T_{H}1, T_{H}2), and lead to a number of events including activation of macrophage-mediated pathogen killing, B cell proliferation, and antibody production. Because of MHC II role in immune response, understanding of MHC II peptide presentation is important in the development of treatment for human pathologies. However, presentation of antigens in the context of mouse MHC II is only somewhat relevant to human disease, since human and mouse MHC complexes recognize antigens differently, e.g., a mouse MHC II may not recognize the same antigens or may present different epitopes than a human MHC II. Thus, the most relevant data for human pathologies is obtained through studying the presentation of antigen epitopes by human MHC II.

Thus, in various embodiments, the genetically engineered animals of the present invention are useful, among other things, for evaluating the capacity of an antigen to initiate an immune response in a human, and for generating a diversity of antigens and identifying a specific antigen that may be used in human vaccine development.

Disclosed herein is a method for determining antigenicity in a human of a peptide sequence, comprising exposing a genetically modified rodent as described herein to a molecule comprising the peptide sequence, allowing the rodent to mount an immune response, and detecting in the rodent a cell that binds a sequence of the peptide presented by a humanized MHC II complex described herein.

Disclosed herein is a method for determining whether a peptide will provoke an immune response in a human, comprising exposing a genetically modified rodent as described herein to the peptide, allowing the rodent to mount an immune response, and detecting in the rodent a cell that binds a sequence of the peptide by a chimeric human/rodent MHC class II molecule as described herein. In one example, the rodent following exposure comprises an MHC class II-restricted CD4+ T cell that binds the peptide.

Disclosed herein is a method for identifying a human CD4+ T cell epitope, comprising exposing a rodent as described herein to an antigen comprising a putative T cell epitope, allowing the rodent to mount an immune response, and identifying the epitope bound by the MHC class II-restricted CD4+ T cell.

Disclosed herein is a method for identifying an antigen that generates a CD4+ T cell response in a human, comprising exposing a putative antigen to a mouse as described herein, allowing the mouse to generate an immune response, detecting a CD4+ T cell response that is specific for the antigen in the context of a human MHC II molecule (e.g., an HLA-DR or HLA-DQ molecule), and identifying the antigen bound by the human MHC II-restricted molecule (e.g., human HLA-DR or HLA-DQ restricted molecule).

In one example, the antigen comprises a bacterial protein. In one embodiment, the antigen comprises a human tumor cell antigen. In one example, the antigen comprises a putative vaccine for use in a human, or another biopharmaceutical. In one example, the antigen comprises a human epitope that generates antibodies in a human. In yet another example, an antigen comprises a yeast or fungal cell antigen. In yet another example, an antigen is derived from a human parasite.

Disclosed herein is a method for determining whether a putative antigen contains an epitope that upon exposure to a human immune system will generate an HLA-DR-restricted immune response (e.g., HLA-DR2- or HLA-DR4-restricted response), comprising exposing a mouse as described herein to the putative antigen and measuring an antigen-specific HLA-DR-restricted (e.g., HLA-DR2- or HLA-DR4-restricted) immune response in the mouse.

Also disclosed herein is a method for determining wherein a putative antigen contains an epitope that upon exposure to a human immune system will generate an HLA-DQ-restricted (e.g., HLA-DQ2- or HLA-DQ8-restricted) immune response.

Also disclosed is a method of generating antibodies to an antigen, e.g., an antigen derived from bacterium, parasite, etc., presented in the context of a human MHC II complex, comprising exposing a mouse described herein to an antigen, allowing a mouse to mount an immune response, wherein the immune response comprises antibody production, and isolating an antibody that recognizes the antigen presented in the context of human MHC II complex. In one example, in order to generate antibodies to the peptide-MHC II, the MHC II humanized mouse is immunized with a peptide-MHC II immunogen.

Disclosed herein is a method for identifying a T cell receptor variable domain that recognizes an antigen presented in the context of MHC II (e.g., human tumor antigen, a vaccine, etc.), comprising exposing a mouse comprising a humanized MHC II complex described herein to the antigen, allowing the mouse to generate an immune response, and isolating from the mouse a nucleic acid sequence encoding a variable domain of a T cell receptor that binds MHC II-restricted antigen. In one example, the antigen is presented in the context of a humanized MHC II (e.g., human HLA II ectodomain/mouse MHC II transmembrane and/or cytoplasmic domain).

The consequence of interaction between a T cell and an APC displaying a peptide in the context of MHC II (e.g., human HLA II ectodomain/mouse MHC II transmembrane and/or cytoplasmic domain) can be measured by a number of techniques known in the art, e.g., T cell proliferation assays, cytokine release assays, etc.

In addition to the ability to identify antigens and their T cell epitopes from pathogens or neoplasms, the genetically modified animals of the invention can be used to identify autoantigens of relevance to human autoimmune disease, and otherwise study human autoimmune disease progression. It is known that polymorphisms within the HLA loci play a role in predisposition to human autoimmune disease. In fact, specific polymorphisms in HLA-DR and HLA-DQ loci have been identified that correlate with development of rheumatoid arthritis, type I diabetes, Hashimoto's thyroiditis, multiple sclerosis, myasthenia gravis, Graves' disease, systemic lupus erythematosus, celiac disease, Crohn's disease, ulcerative colitis, and other autoimmune disorders. See, e.g., Wong and Wen (2004) What can the HLA transgenic mouse tell us about autoimmune diabetes?, Diabetologia 47:1476-87; Taneja and David (1998) HLA Transgenic Mice as Humanized Mouse Models of Disease and Immunity, J. Clin. Invest. 101:921-26; Bakker et al. (2006), *supra;* and International MHC and Autoimmunity Genetics Network (2009) Mapping of multiple susceptibility variants within the MHC region for 7 immune-mediated diseases, Proc. Natl. Acad. Sci. USA 106:18680-85.

Thus, the methods of making a humanized MHC II complex rodents described herein can be used to introduce MHC II molecules thought to be associated with specific human autoimmune diseases, and progression of human autoimmune disease can be studied. In addition, rodents described herein can be used to develop animal models of human autoimmune disease. Mice according to the invention carrying humanized MHC II proteins described herein can be used to identify potential autoantigens, to map epitopes involved in disease progression, and to design strategies for autoimmune disease modulation.

In addition, the genetically modified rodents described herein may be used in the study of human allergic response. As allergic responses appear to be associated with MHC II alleles, genetically modified animals described herein may be used to determine HLA restriction of allergen specific T cell response and to develop strategies to combat allergic response.

In addition, the genetically modified animals of the invention and the humanized HLA molecules expressed by the same can be used to test antibodies that block antigen presentation by human HLA molecules associated with human disease progression. Thus, disclosed herein is a method of determining whether an antibody is capable of blocking presentation of an antigen by an HLA molecule linked to a human disease, e.g., a human disease described above, comprising exposing a cell expressing a humanized HLA described herein to a test antibody and determining whether the test antibody is capable of blocking the presentation of the antigen by a humanized HLA to immune cells (e.g., to T cells), e.g., by measuring its ability to block humanized HLA-restricted immune response. In one example, the method is conducted in an animal expressing the humanized HLA, e.g., an animal expressing disease-associated humanized HLA that serves as a disease model for the disease.

### EXAMPLES

The invention will be further illustrated by the following nonlimiting examples. These Examples are set forth to aid in the understanding of the invention but are not intended to, and should not be construed to, limit its scope in any way. The Examples do not include detailed descriptions of conventional methods that would be well known to those of ordinary skill in the art (molecular cloning techniques, etc.). Unless indicated otherwise, parts are parts by weight, molecular weight is average molecular weight, temperature is indicated in Celsius, and pressure is at or near atmospheric.

### Example 1. Deletion of the Endogenous MHC class II H-2A and H-2E Loci

The targeting vector for introducing a deletion of the endogenous MHC class II H-2Ab1, H-2Aa, H-2Eb1, H-2Eb2, and H-2Ea genes was made using VELOCIGENE^{®} genetic engineering technology (see, *e.g.,* US Pat. No. 6,586,251 and Valenzuela et al., *supra).* Bacterial Artificial Chromosome (BAC) RP23-458i22 (Invitrogen) DNA was modified to delete the endogenous MHC class II genes H-2Ab1, H-2Aa, H-2Eb1, H-2Eb2, and H-2Ea.

Briefly, upstream and downstream homology arms were derived by PCR of mouse BAC DNA from locations 5' of the H-2Ab2 gene and 3' of the H-2Ea gene, respectively. As depicted in FIG. 5, these homology arms were used to make a cassette that deleted ~79 kb of RP23-458i22 comprising genes H-2Ab1, H-2Aa, H-2Eb1, H-2Eb2, and H-2Ea of the MHC class II locus by bacterial homologous recombination (BHR). This region was replaced with a hygromycin cassette flanked by lox66 and lox71 sites. The final targeting vector from 5' to 3' included a 34 kb homology arm comprising mouse genomic sequence 5' to the H-2Ab2 gene of the endogenous MHC class II locus, a 5' lox66 site, a hygromycin cassette, a 3' lox71 site and a 63 kb homology arm comprising mouse genomic sequence 3' to the H-2Ea gene of the endogenous MHC class II locus (MAID 5111, see FIG. 5).

The BAC DNA targeting vector (described above) was used to electroporate mouse ES cells to create modified ES cells comprising a deletion of the endogenous MHC class II locus. Positive ES cells containing a deleted endogenous MHC class II locus were identified by the quantitative PCR assay using TAQMAN^{™} probes (Lie and Petropoulos (1998) Curr. Opin. Biotechnology 9:43-48). The upstream region of the deleted locus was confirmed by PCR using primers 5111U F (CAGAACGCCAGGCTGTAAC; SEQ ID NO:1) and 5111U R (GGAGAGCAGGGTCAGTCAAC; SEQ ID NO:2) and probe 5111U P (CACCGCCACTCACAGCTCCTTACA; SEQ ID NO:3), whereas the downstream region of the deleted locus was confirmed using primers 5111D F (GTGGGCACCATCTTCATCATTC; SEQ ID NO:4) and 5111D R (CTTCCTTTCCAGGGTGTGACTC; SEQ ID NO:5) and probe 5111D P (AGGCCTGCGATCAGGTGGCACCT; SEQ ID NO:6). The presence of the hygromycin cassette from the targeting vector was confirmed using primers HYGF (TGCGGCCGATCTTAGCC; SEQ ID NO:7) and HYGR (TTGACCGATTCCTTGCGG; SEQ ID NO:8) and probe HYGP (ACGAGCGGGTTCGGCCCATTC; SEQ ID NO:9). The nucleotide sequence across the upstream deletion point (SEQ ID NO:10) included the following, which indicates endogenous mouse sequence upstream of the deletion point (contained within the parentheses below) linked contiguously to cassette sequence present at the deletion point: (TTTGTAAACA AAGTCTACCC AGAGACAGAT GACAGACTTC AGCTCCAATG CTGATTGGTT CCTCACTTGG GACCAACCCT) CTCGAGTACC GTTCGTATAA TGTATGCTAT ACGAAGTTAT ATGCATCCGG GTAGGGGAGG. The nucleotide sequence across the downstream deletion point (SEQ ID NO:11) included the following, which indicates cassette sequence contiguous with endogenous mouse sequence downstream of the deletion point (contained within the parentheses below): CCTCGACCTG CAGCCCTAGG ATAACTTCGT ATAATGTATG CTATACGAAC GGTAGAGCTC (CACAGGCATT TGGGTGGGCA GGGATGGACG GTGACTGGGA CAATCGGGAT GGAAGAGCAT AGAATGGGAG TTAGGGAAGA). Positive ES cell clones were then used to implant female mice using the VELOCIMOUSE^{®} method (described below) to generate a litter of pups containing a deletion of the endogenous MHC class II locus.

Targeted ES cells described above were used as donor ES cells and introduced into an 8-cell stage mouse embryo by the VELOCIMOUSE^{®} method (see, *e.g.,* US Pat. No. 7,294,754 and Poueymirou et al. (2007) F0 generation mice that are essentially fully derived from the donor gene-targeted ES cells allowing immediate phenotypic analyses, Nature Biotech. 25(1):91-99). Mice bearing a deletion of H-2Ab1, H-2Aa, H-2Eb1, H-2Eb2, and H-2Ea genes in the endogenous MHC class II locus were identified by genotyping using a modification of allele assay (Valenzuela *et al., supra*) that detected the presence of the hygromycin cassette and confirmed the absence of endogenous MHC class II sequences.

Mice bearing a deletion of H-2Ab1, H-2Aa, H-2Eb1, H-2Eb2, and H-2Ea genes in the endogenous MHC class II locus can be bred to a Cre deletor mouse strain (see, e.g., International Patent Application Publication No. WO 2009/114400) in order to remove any loxed hygromycin cassette introduced by the targeting vector that is not removed, e.g., at the ES cell stage or in the embryo. Optionally, the hygromycin cassette is retained in the mice.

### Example 2. Generation of Large Targeting Vector (LTVEC) Comprising Chimeric HLA-DR4/H-2E Gene

A targeting vector to introduce humanized MHC II sequences was designed as depicted in FIG. 4. Using VELOCIGENE^{®} genetic engineering technology, Bacterial Artificial Chromosome (BAC) RP23-458i22 DNA was modified in various steps to: (1) create a vector comprising a functional I-E α exon 1 from BALB/c H-2Ea gene (FIG. 4A); (2) create a vector comprising replacement of exons 2 and 3 of mouse I-E β gene with those of human DRβ1^{∗}04 and replacement of exons 2 and 3 of mouse I-E α with those of human DRα1^{∗}01 (FIGs. 4B); (3) create a vector carrying exons 2 and 3 of human DRβ1^{∗}04 amongst remaining mouse I-E β exons, and exons 2 and 3 of human DRα1^{∗}01 amongst remaining mouse I-E α exons including a functional I-E α exon 1 from BALB/c mouse (step (1) (FIG. 4C); and (4) remove a cryptic splice site in the vector generated in (3) (FIG. 4D).

Specifically, because in the C57Bl/6 mice, the I-E α gene is a pseudogene due to the presence of a non-functional exon 1, first, a vector comprising a functional I-E α exon 1 from BALB/c H-2Ea gene was created (FIG. 4A). RP23-458i22 BAC was modified by bacterial homologous recombination (1.BHR) to replace chloramphenicol resistance gene with that of spectromycin. The resultant vector was further modified by BHR to replace the entire I-A and I-E coding region with a neomycin cassette flanked by recombination sites (2.BHR). Another round of BHR (3. BHR) with the construct comprising an exon encoding BALB/c I-Eα leader (exon 1) and chloramphenicol gene flanked by PI-SceI and I-CeuI restriction sites resulted in a vector comprising a functional BALB/c H-2Ea exon 1.

Independently, in order to generate a vector comprising replacement of exons 2 and 3 of mouse I-E β gene with those of human DRβ1^{∗}04 and replacement of exons 2 and 3 of mouse I-E α with those of human DRα1^{∗}01, RP23-458i22 BAC was modified via several homologous recombination steps, 4. BHR - 8. BHR (FIG. 4B). The resultant nucleic acid sequence was flanked by PI-SceI/I-CeuI restriction sites to allow ligation into the construct carrying BALB/c I-Eα exon 1, mentioned above (FIG. 4C).

The sequence of the final construct depicted in FIG. 4C contained a cryptic splice site at the 3' end of the BALB/c intron. Several BHR steps (11. BHR - 12. BHR) followed by a deletion step were performed to obtain the final targeting vector (MAID 1680) that was used to electroporate into ES cells (FIG. 4D).

In detail, the final targeting vector (MAID 1680), from 5' to 3', was comprised of a 5' mouse homology arm consisting of ~26 kb of mouse genomic sequence ending just upstream of the H-2Ab1 gene of the endogenous MHC class II locus; an ~59 kb insert containing the humanized MHC II β chain gene (humanized H-2Eb1 gene) and humanized MHC II α chain gene (humanized H-2Ea gene) and a floxed neomycin cassette; and a 3' mouse homology arm consisting of ~57 kb of mouse genomic sequence beginning just downstream of the H-2Ea gene of the endogenous MHC class II locus. The nucleotide sequence across the junction between the 5' arm and the insert (SEQ ID NO:12) included the following: (TGCTGATTGG TTCCTCACTT GGGACCAACC C) TAAGCTTTA *TCTATGTCGG GTGCGGAGAA AGAGGTAATG AAATGGCACA* AGGAGATCAC ACACCCAAAC CAAACTCGCC, where the italicized sequence is a unique PI-SceI site, and mouse genomic sequence in the 5' homology arm is in parentheses. The nucleotide sequence across the junction between the insert and the 3' arm (SEQ ID NO:13) included the following: CACATCAGTG AGGCTAGAAT AAATTAAAAT CGCTAATATG AAAATGGGG (ATTTGTACCT CTGAGTGTGA AGGCTGGGAA GACTGCTTTC AAGGGAC), where the mouse genomic sequence in the 3' homology arm is in parentheses.

Within the ~59 kb insert, the H-2Eb1 gene was modified as follows: a 5136 bp region of H-2Eb1, including the last 153 bp of intron1, exon 2, intron 2, exon 3, and the first 122 bp of intron 3, was replaced with the 3111 bp homologous region of human HLA-DRB1^{∗}04, including the last 148 bp of intron 1, exon 2, intron 2, exon 3, and the first 132 bp of intron 3. At the junction between the human and mouse sequences of intron 3, a cassette consisting of a 5' lox2372 site, UbC promoter, neomycin resistance gene, and a 3' lox2372 site, was inserted. The resulting gene encoded a chimeric HLA-DRB1∗04/H-2Eb1 protein comprised of the mouse H-2Eb1 leader, the human β1 and β2 domains from DRB1^{∗}04, and the mouse transmembrane domain and cytoplasmic tail. The nucleotide sequence across the mouse/human junction in intron 1 (SEQ ID NO:14) included the following: (TCCATCACTT CACTGGGTAG CACAGCTGTA ACTGTCCAGC CTG) *GGTACCGAGC TCGGATCCAC TAGTAACGGC CGCCAGTGTG CTGGAATTC GCCCTTGATC GAGCTCCCTG* GGCTGCAGGT GGTGGGCGTT GCGGGTGGGG CCGGTTAA, where the italicized sequence is a multiple cloning site introduced during the cloning steps, and the mouse intron 1 sequences are in parentheses. The nucleotide sequence across the junction between the human intron 3 and neomycin cassette (SEQ ID NO:15) included the following: (ATCTCCATCA GAAGGGCACC GGT) *ATAACTT CGTATAAGGTATCCTATACG AAGTTATATG* CATGGCCTCC GCGCCGGGTT, where the 5' lox2372 site is italicized, and human intron 3 sequence is in parentheses. The nucleotide sequence across the junction between the neomycin cassette and mouse intron 3 (SEQ ID NO:16) included the following: *ATAACTTCGT ATAAGGTATC CTATACGAAG* TTA TCTCGAG (TGGCTTACAG GTAGGTGCGT GAAGCTTCTA CAAGCACAGT TGCCCCCTGG), where the 3' lox2372 site is italicized, and the mouse intron 3 sequence is in parentheses.

Also within the ~59 kb insert, the H-2Ea gene was modified as follows: a 1185 bp region of H-2Ea, including the last 101 bp of intron1, exon 2, intron 2, exon 3, and the first 66 bp of intron 3, was replaced with the 1189 bp homologous region of human HLA-DRA1^{∗}01, including the last 104 bp of intron 1, exon 2, intron 2, exon 3, and the first 66 bp of intron 3. As described above, because exon 1 of the C57BL/6 allele of H-2Ea contains a deletion which renders the gene nonfunctional, H-2Ea exon 1 and the remainder of intron 1 were replaced with the equivalent 2616 bp region from the BALB/c allele of H-2Ea, which is functional. The resulting gene encoded a chimeric H-2Ea/HLA-DRA1^{∗}01 protein comprised of the mouse H-2Ea leader from BALB/c, the human α1 and α2 domains from DRA1^{∗}01, and the mouse transmembrane domain and cytoplasmic tail. The nucleotide sequence across the mouse/human junction in intron 1 (SEQ ID NO:17) included the following: (CTGTTTCTTC CCTAACTCCC ATTCTATGCT CTTCCATCCC GA) *CCGCGGCCCA* ATCTCTCTCC ACTACTTCCT GCCTACATGT ATGTAGGT, where the italicized sequence is a restriction enzyme site introduced during the cloning steps, and the BALB/c intron 1 sequences are in parentheses. The nucleotide sequence across the human/mouse junction in intron 3 (SEQ ID NO:18) included the following: CAAGGTTTCC TCCTATGATG CTTGTGTGAA ACTCGG*GGCC GGCC* (AGCATTTAAC AGTACAGGGA TGGGAGCACA GCTCAC), where the italicized sequence is a restriction enzyme site introduced during the cloning steps, and the mouse intron 3 sequences are in parentheses. The nucleotide sequence across the C57BL/6-BALB/c junction 5' of exon 1 (SEQ ID NO:19) included the following: (GAAAGCAGTC TTCCCAGCCT TCACACTCAG AGGTACAAAT) CCCCATTTTC ATATTAGCGA TTTTAATTTA TTCTAGCCTC, where the C57BL/6-specific sequences are in parentheses. The nucleotide sequence across the BALB/c-C57BL/6 junction 3' of exon 1 (SEQ ID NO:20) included the following: TCTTCCCTAA CTCCCATTCT ATGCTCTTCC ATCCCGA *CCG CGG* (CCCAATC TCTCTCCACT ACTTCCTGCC TACATGTATG), where SacII restriction site is italicized, and C57BL/6 sequences are in parenthesis.

### Example 3. Generation of Genetically Modified HLA-DR4 Mice

Simplified diagrams of the strategy for generating humanized MHC II mice using the vector of Example 2 are presented in FIGs. 5 and 8.

Specifically, MAID1680 BAC DNA (described above) was used to electroporate MAID5111 ES cells to create modified ES cells comprising a replacement of the endogenous mouse I-A and I-E loci with a genomic fragment comprising a chimeric human DR4/mouse I-E locus. Positive ES cells containing deleted endogenous I-A and I-E loci replaced by a genomic fragment comprising a chimeric human DR4/mouse I-E locus were identified by a quantitative PCR assay using TAQMAN^{™} probes (Lie and Petropoulos, *supra).* The insertion of the human DRα sequences was confirmed by PCR using primers hDRAlF (CTGGCGGCTTGAAGAATTTGG; SEQ ID NO:21), hDRAIR (CATGATTTCCAGGTTGGCTTTGTC; SEQ ID NO:22), and probe hDRA1P (CGATTTGCCAGCTTTGAGGCTCAAGG; SEQ ID NO:23). The insertion of the human DRβ sequences was confirmed by PCR using primers hDRBIF (AGGCTTGGGTGCTCCACTTG; SEQ ID NO:24), hDRBIR (GACCCTGGTGATGCTGGAAAC; SEQ ID NO:25), and probe hDRBIP (CAGGTGTAAACCTCTCCACTCCGAGGA; SEQ ID NO:26).The loss of the hygromycin cassette from the targeting vector was confirmed with primers HYGF (TGCGGCCGATCTTAGCC; SEQ ID NO:7) and HYGR (TTGACCGATTCCTTGCGG; SEQ ID NO:8) and probe HYGP (ACGAGCGGGTTCGGCCCATTC; SEQ ID NO:9).

Positive ES cell clones were then used to implant female mice using the VELOCIMOUSE^{®} method (*supra*) to generate a litter of pups containing a replacement of the endogenous I-A and I-E loci with a chimeric human DR4/mouse I-E locus. Targeted ES cells described above were used as donor ES cells and introduced into an 8-cell stage mouse embryo by the VELOCIMOUSE^{®} method. Mice bearing a chimeric human DR4/mouse I-E locus were identified by genotyping using a modification of allele assay (Valenzuela et al., *supra)* that detected the presence of a chimeric human DR4/mouse I-E locus.

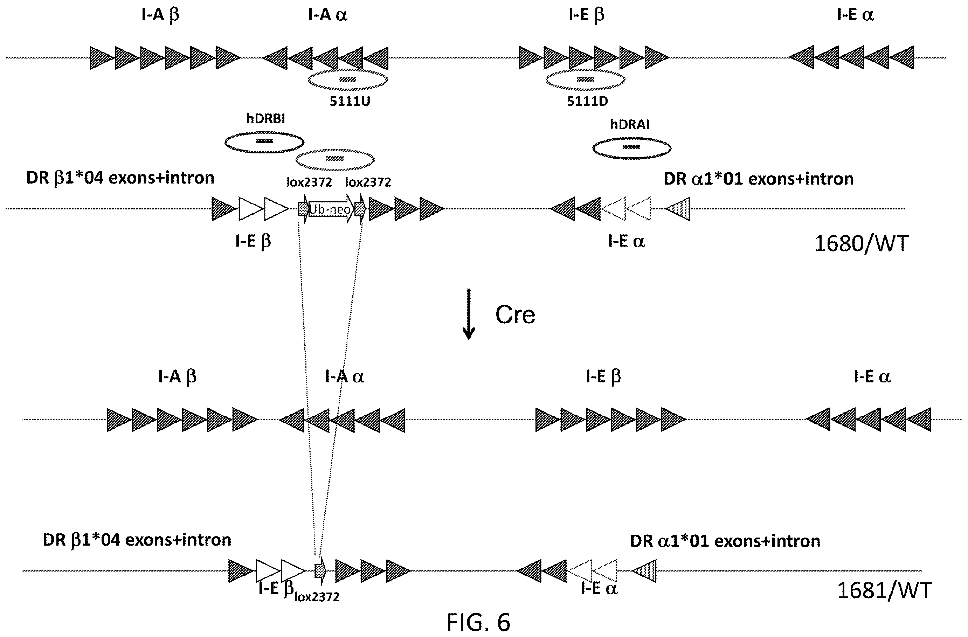

Mice bearing a chimeric human DR4/mouse I-E locus can be bred to a Cre deletor mouse strain (see, e.g., International Patent Application Publication No. WO 2009/114400) in order to remove any loxed neomycin cassette introduced by the targeting vector that is not removed, e.g., at the ES cell stage or in the embryo (See FIG. 6).

### Example 4. Expression of the Chimeric HLA-DR4/H-2E in Genetically Modified Mice

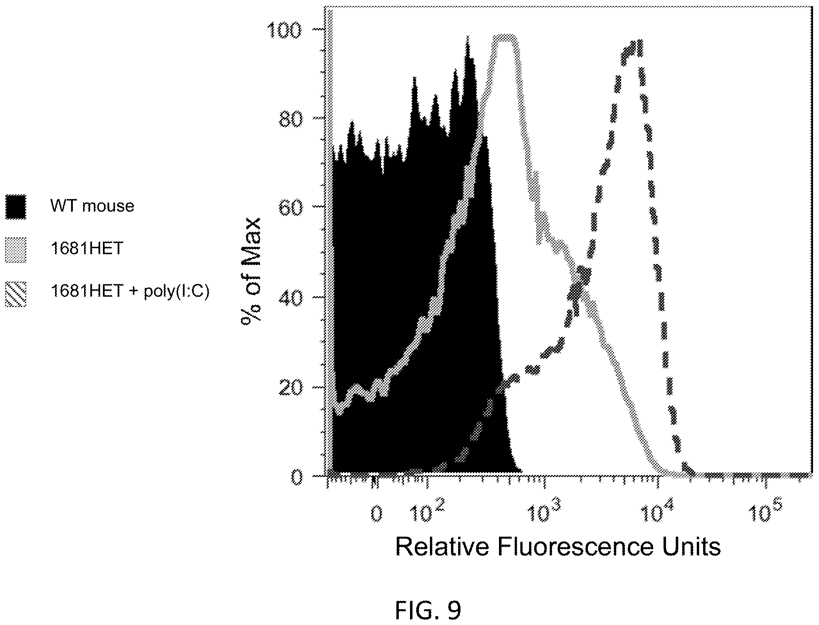

Spleens from WT or heterozygous humanized HLA-DR4 mice ("1681 HET") were perfused with Collagenase D (Roche Bioscience) and erythrocytes were lysed with ACK lysis buffer. Splenocytes were cultured for two days with 25 micrograms/mL poly(I:C) to stimulate the expression of MHC-II genes. Cell surface expression of human HLA-DR4 was analyzed by FACS using fluorochrome-conjugated anti-CD3 (17A2), anti-CD19 (1D3), anti-CD11c (N418), anti-F480 (BM8), anti-I-A/I-E (M15) and anti-HLADR (L243). Flow cytometry was performed using BD-LSRII. Expression of human HLA-DR4 was clearly detectable on the surface of CD19+ B cells and was significantly upregulated upon stimulation by toll-like receptor agonist poly(I:C) (see FIG. 9).

### Example 5. Genetically Modified HLA-DQ2.5, HLA-DQ8, and HLA-DR2 Mice

Mice harboring chimeric human HLA-DQ2.5/H-2A, HLA-DQ8/H-2A and HLA-DR2/H-2E were made using VELOCIGENE^{®} genetic engineering technology (see, *e.g.,* US Pat. No. 6,586,251 and Valenzuela et al., *supra).* LTVECs harboring humanized genes were generated by gene synthesis and modification of BACs by bacterial homologous recombination and digestion/ligation techniques.

Human HLA-DQ2.5, -DQ8.1, and DR2 α and β chain sequences were synthesized by Blue Heron Gene Synthesis Company (WA, USA) based on publically available gene sequences. Specifically, synthesized human HLA-DQ2.5 α (DQA1^{∗}05) and β (DQB1^{∗}02) chains and mouse BAC RP23-444J20 were modified and used to generate an LTVEC harboring chimeric HLA-DQ2.5/H-2A, depicted in FIG.10A. Similarly, synthesized human HLA-DQ8.1 α (DQA1^{∗}0301) and β (DQB1^{∗}0302) chains and mouse BAC RP23-444J20 were modified and used to generate an LTVEC harboring chimeric HLA-DQ8.1/H-2A, depicted in FIG. 10B. For HLA-DR2/H-2E, synthesized human HLA-DR2 β chain (DRB1^{∗}1501) was used to generate a vector comprising DRβ1^{∗}02(1501) exons and intron, and swapped using bacterial homologous recombination into the LTVEC comprising chimeric HLA-DR4/H-2E gene (MAID 1680 of Example 2 above). The resulting HLA-DR2/H-2E LTVEC is depicted in FIG. 10C. The various nucleotide sequence junctions of the resulting LTVECs (e.g., mouse/human sequence junctions, human/mouse sequence junctions, or junctions of mouse or human sequence with selection cassettes) are summarized below in Table 1 and listed in the Sequence Listing; their locations are indicated in the schematic diagram of FIG. 10. In Table 1 below, the mouse sequences are in regular font; the human sequences are in parentheses; the Lox sequences are italicized; and the restriction sites introduced during cloning steps and other vector-based sequences (e.g., multiple cloning sites, etc.) are bolded. The nucleotide sequence junctions for the humanized H-2Ea in HLA-DR2/H-2E are the same as previously described for HLA-DR4/H-2E in Example 2 above (SEQ ID NOs:17-20).

**Table 1: Junctions of LTVECs for HLA-DR2/H-2E, HLA-DQ2.5/H-2A, and HLA-DQ8/H-2A Mice**

| **SEQ ID NO** | **Nucleotide Sequence** |
|---|---|
| **27** | GAAGAGACTGCCCGGCCCGAGGCCCCGAGGCCCGACGGTCCCCAGAGAGC (GTGGTCGCGCGGGCTGTTCCACAGCTCCGGGCCGGGTCAGGGTGGCGGCT) |
| **28** | (AGAGCTCCTTCTGACCCATCCCTTCCCATCTCTTATCCCTGATGTCACTG) ACCGGT ATAACTTCGTATAATGTATGCTATACGAAGTTAT ATGCATGGCC |
| **29** | CGCC ATAACTTCGTATAATGTATGCTATACGAAGTTAT GTCGACCTCGAG CTATATGATCTGCTTCCACTACTGAGCTGAGACCTACAGGAAATCATATC |
| **30** | GTGGGTTGGGCTTATGGAATGACATGAAATCTAGCTTTAGGAGCTTTTTA (GAAGTGTGGAAAACAAGTTTTGGGATATAGGAGTAAAAGGCAGGAAGTTC) |
| **31** | (CCTTAATACACAAAATGATGAAAGAAACTGACTTTCAAATATTACGGGCT) CTCGAGCGGCCGCCAGTGTGATGGATGGTACCTAACTATAACGGTCCT |
| | **AAGGTAGCGAC** CATTTTTGGGGTTTGTTTCTACCACTCATTCCTCCCT CCCACTTGGTAT |
| **32** | CCCAATTCCCAATTTCTTCCTTCCCTCACTGGGCCCACTAGTAGAATCAT GGTTTGGTGGATTGTGAGCTGAC GGCCGGCC CAACCAGAGCCTCCTGAAGAATGGATCACACAGTTGTTAGACCTCTTCATA TTGAGAATTTGTATCTCCCTCTTCTGTGGGTTTCCCATTGCCCGGCTCCAG GCATCCCTCAGCTGCACAACTCAC (CCACAATGTCTTCACCTCCACAG) AGGCTGAGCATGGTGGTCAGGGCGAGGACCCCCAGAATCAGAGCTCTGCT |
| **33** | CGGCTGCTGTGGTGGTGCTGATGGTGCTGAGCAGCCCAGGGACTGAGGGC (AGAGACTCTCCCGGTAAGTGCAGGGCCACTGCTCTCCAGAGCCGCCACTC) |
| **34** | (GAGCTCCTTCTGACCCATTCCTTCCCATCTCTTATCCCTGATGTCACTAC) CTCGAG ATAACTTCGTATAATGTATGCTATACGAAGTTAT ATGCATGGCC |
| **35** | TACGAAGTTAT ACGCGTAACTATAACGGTCCTAAGGTAGCGAGCGGCCGC CTATATGATCTGCTTCCACTACTGAGCTGAGACCTACAGGAAATCATATC |
| **36** | AGGGTGAGGGGGCAAGTTAATCTACATAGCCTTTGTGGGTTGGGCTTATG GGCGCGCCATTCGCTACCTTAGGACCGTTATAGTTA (TGAAGTGTGGAAAACAAGTTTTGGGATATAGAGTAAAAGGCAGGAAGTTC) |
| **37** | (TTCAGCTCCAGAGAACATTCCTCACTCATGCACTCACCCACAATGTCTTC) ACCTCCACAGAGGCTGAGCATGGTGGTCAGGGCGAGGACCCCCAGAATCA |
| **38** | GAGTTCCTCCATCACTTCACTGGGTAGCACAGCTGTAACTGTCCAGCCTG (TCCTGGGCTGCAGGTGGTGGGCGTTGCGGGTGGGGCCGGTTAAGGTTCCA) |
| **39** | (TCCCACATCCTATTTTAATTTGCTCCATGTTCTCATCTCCATCAGCACAG) |
| | CTCGAG ATAACTTCGTATAATGTATGCTATACGAAGTTAT ATGCATGGCC |
| **40** | ATACGAAGTTAT GCTAGTAACTATAACGGTCCTAAGGTAGCGAGTGGCTT ACAGGTAGGTGCGTGAAGCTTCTACAAGCACAGTTGCCCCCTGGGAAGCA |

The large targeting vectors are used to electroporate MAID5111 ES cells to create modified ES cells comprising a replacement of the endogenous mouse I-A and I-E loci with genomic fragments comprising a chimeric HLA-DQ2.5/H-2A, HLA-DQ8/H-2A, or HLA-DR2/H-2E. Positive ES cells containing deleted endogenous I-A and I-E loci replaced by genomic fragments comprising chimeric loci are identified by a quantitative PCR assay using TAQMAN^{™} probes (Lie and Petropoulos, *supra*).

Positive ES cell clones are then used to implant female mice using the VELOCIMOUSE^{®} method (*supra*) to generate a litter of pups containing a replacement of the endogenous I-A and I-E loci with a chimeric human/mouse locus. Targeted ES cells are used as donor ES cells and introduced into an 8-cell stage mouse embryo by the VELOCIMOUSE^{®} method. Mice bearing a chimeric human/mouse locus are identified by genotyping using a modification of allele assay (Valenzuela et al., *supra)* that detects the presence of a chimeric human/mouse locus.

Mice bearing a chimeric human/mouse locus are bred to a Cre deletor mouse strain (see, e.g., International Patent Application Publication No. WO 2009/114400) in order to remove any *lox*ed neomycin or hygromycin cassette introduced by the targeting vector that is not removed, e.g., at the ES cell stage or in the embryo.

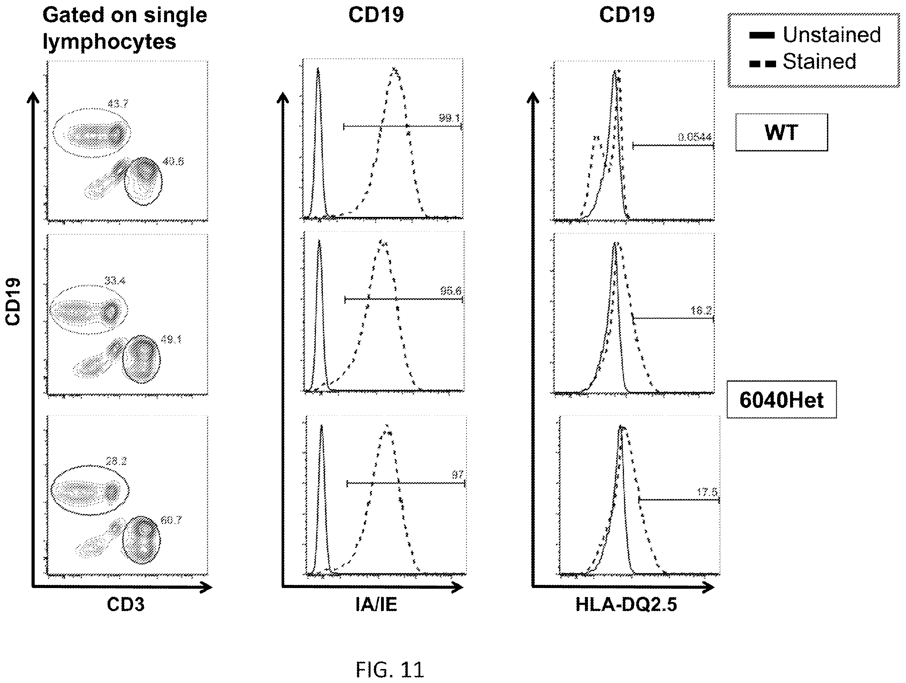

Expression of chimeric human/mouse protein is tested. Specifically, for HLA-DQ2.5/H-2A genetically modified animals, blood from WT or heterozygous humanized HLA-DQ2.5 mice ("6040 HET"; cassette deleted) were drawn into heparin coated capillary tubes and erythrocytes were lysed with ACK lysis buffer. Cell surface expression of human HLA-DQ2.5 was analyzed by FACS using fluorochrome-conjugated anti-CD3 (17A2), anti-CD19 (1D3), anti-I-A/I-E (M15) and anti-HLA-DQ (Tu169) antibodies. Flow cytometry was performed using BD-Fortessa. Expression of both mouse MHC-II (I-A/I-E) and human HLA-DQ2.5 was clearly detectable on the surface of antigen presenting CD19+ mouse B cells (FIG. 11).

### Equivalents

Those skilled in the art will recognize, or be able to ascertain using no more than routine experimentation, many equivalents of the specific embodiments of the invention described herein. Such equivalents are intended to be encompassed by the following claims.

## Claims

1. A rodent comprising
(A) at a first endogenous MHC II locus, a nucleotide sequence encoding a first chimeric human/rodent MHC II α polypeptide and a nucleotide sequence encoding a first chimeric human/rodent MHC II β polypeptide,
wherein the first chimeric human/rodent MHC II α polypeptide comprises α1 and α2 domains of a human HLA-DR α polypeptide operably linked to transmembrane and cytoplasmic domains of an endogenous rodent MHC II α polypeptide,
wherein the first chimeric human/rodent MHC II β polypeptide comprises β1 and
β2 domains of a human HLA-DR β polypeptide operably linked to transmembrane and cytoplasmic domains of an endogenous rodent MHC II β polypeptide, and
wherein the first chimeric human/rodent MHC II α polypeptide and the first chimeric human/rodent MHC II β polypeptide form a first MHC II complex on a surface of a cell of the rodent; and
(B) at a second endogenous MHC II locus, a nucleotide sequence encoding a second chimeric human/rodent MHC II α polypeptide and a nucleotide sequence encoding a second chimeric human/rodent MHC II β polypeptide,
wherein the second chimeric human/rodent MHC II α polypeptide comprises α1 and α2 domains of a human HLA-DQ α polypeptide operably linked to transmembrane and cytoplasmic domains of an endogenous rodent MHC II α polypeptide,
wherein the second chimeric human/rodent MHC II β polypeptide comprises β1 and β2 domains of a human HLA-DQ β polypeptide operably linked to transmembrane and cytoplasmic domains of an endogenous rodent MHC II β polypeptide,
wherein the second chimeric human/rodent MHC II α polypeptide and the second chimeric human/rodent MHC II β polypeptide form a second MHC II complex on a surface of a cell of the rodent, and
wherein (i) the HLA-DR α polypeptide is an HLA-DR2 α polypeptide and the HLA-DR β polypeptide is an HLA-DR2 β polypeptide, or (ii) the HLA-DR α polypeptide is an HLA-DR4 α polypeptide, the HLA-DR β polypeptide is an HLA-DR4 β polypeptide, the HLA-DQ α polypeptide is an HLA-DQ2 or HLA-DQ8 α polypeptide and the HLA-DQ β polypeptide is an HLA-DQ2 or HLA-DQ8 β polypeptide.

2. The rodent of claim 1, wherein the nucleotide sequence encoding the first chimeric human/rodent MHC II α polypeptide and the nucleotide sequence encoding the second chimeric human/rodent MHC II α polypeptide are operably linked to endogenous rodent MHC II α promoter and regulatory elements, and the nucleotide sequence encoding the first chimeric human/rodent MHC II β polypeptide and the nucleotide sequence encoding the second chimeric human/rodent MHC II β polypeptide are operably linked to endogenous rodent MHC II β promoter and regulatory elements.

3. The rodent of claim 1, wherein the rodent does not express a functional endogenous MHC II polypeptide from the first endogenous MHC II locus or from the second endogenous MHC II locus.

4. The rodent of any one of claims 1-3, wherein the rodent is a rat.

5. The rodent of any one of claims 1-3, wherein the rodent is a mouse.

6. The rodent of claim 5, wherein the first endogenous MHC II locus is an endogenous H-2E locus and wherein the first chimeric human/rodent MHC II α polypeptide comprises the α1 and α2 domains of a human HLA-DR2 or HLA-DR4 polypeptide operably linked to a transmembrane domain and a cytoplasmic domain of a mouse H-2E α polypeptide and the first chimeric human/rodent MHC II β polypeptide comprises the β1 and β2 domains of the human HLA-DR2 or HLA-DR4 β polypeptide operably linked to a transmembrane domain and a cytoplasmic domain from a mouse H-2E β polypeptide.

7. The rodent of claim 5, wherein the second endogenous MHC II locus is an endogenous H-2A locus and wherein the second chimeric human/rodent MHC II α polypeptide comprises the α1 and α2 domains of a human HLA-DQ2 or HLA-DQ8 α polypeptide operably linked to a transmembrane domain and a cytoplasmic domain from a mouse H-2A α polypeptide and the second chimeric human/rodent MHC II β polypeptide comprises the β1 and β2 domains of a human HLA-DQ2 or HLA-DQ8 β polypeptide operably linked to a transmembrane domain and a cytoplasmic domain of a mouse H-2A β polypeptide.

8. The rodent of claim 6, wherein the endogenous H-2E locus lacks an endogenous nucleotide sequence encoding α1 and α2 domains of a mouse H-2E α polypeptide, and the endogenous H-2E locus lacks an endogenous nucleotide sequence encoding β1 and β2 domains of a mouse H-2E β polypeptide.

9. The rodent of claim 7, wherein the endogenous H-2A locus lacks an endogenous nucleotide sequence encoding α1 and α2 domains of a mouse H-2A α polypeptide, and the endogenous H-2A locus lacks an endogenous nucleotide sequence encoding β1 and β2 domains of a mouse H-2A β polypeptide.

10. The rodent of any one of claims 1-3 or claims 5-7, wherein the rodent is a mouse, and wherein:
(a) the first endogenous MHC II locus is an endogenous MHC II H-2E locus, the first chimeric human/rodent MHC II α polypeptide is a chimeric human/rodent HLA-DR2/H-2E MHC II α polypeptide and the first chimeric human/rodent MHC II β polypeptide is a chimeric human/rodent HLA-DR2/H-2E MHC II β polypeptide; and
(b) the second endogenous MHC II locus is an endogenous MHC II H-2A locus, the second chimeric human/rodent MHC II α polypeptide is a chimeric human/rodent HLA-DQ8.1/H-2A MHC II α polypeptide and the second chimeric human/rodent MHC II β polypeptide is a chimeric human/rodent HLA-DQ8.1/H-2A MHC II β polypeptide,
wherein the mouse expresses a chimeric human/mouse HLA-DR2/H-2E MHC II complex and a chimeric human/mouse HLA-DQ8.1/H-2A MHC II complex on a surface of a cell of the mouse.

11. A method of modifying endogenous MHC II loci in a mouse ES cell, wherein the method comprises:
replacing at a first endogenous MHC II locus in the ES cell:
at least a nucleotide sequence encoding α1 and α2 domains of a first mouse MHC II α polypeptide with at least a nucleotide sequence encoding α1 and α2 domains of a first human MHC II α polypeptide and
at least a nucleotide sequence encoding β1 and β2 domains of a first mouse MHC II β polypeptide with at least a nucleotide sequence encoding β1 and β2 domains of a first human MHC II β polypeptide
to generate a first modified chimeric MHC II locus encoding α1 and α2 domains of the first human MHC II α polypeptide operably linked to transmembrane and cytoplasmic domains of the first mouse MHC II α polypeptide and β1 and β2 domains of the first human MHC II β polypeptide operably linked to transmembrane and cytoplasmic domains of the first mouse MHC II β polypeptide; and
replacing at a second endogenous MHC II locus in the ES cell:
at least a nucleotide sequence encoding α1 and α2 domains of a second mouse MHC II α polypeptide with at least a nucleotide sequence encoding α1 and α2 domains of a second human MHC II α polypeptide and
at least a nucleotide sequence encoding β1 and β2 domains of a second mouse MHC II β polypeptide with at least a nucleotide sequence encoding β1 and β2 domains of a second human MHC II β polypeptide
to generate a second modified chimeric MHC II locus encoding α1 and α2 domains of the second human MHC II α polypeptide operably linked to transmembrane and cytoplasmic domains of the second mouse MHC II α polypeptide and β1 and β2 domains of the second human MHC II β polypeptide operably linked to transmembrane and cytoplasmic domains of the second mouse MHC II β polypeptide, and
wherein the first human MHC II α polypeptide and the second human MHC II α polypeptide are each selected from the group consisting of an α chain of HLA-DR2, HLA-DR4, HLA-DQ2, HLA-DQ2.5, HLA-DQ8, and HLA-DQ8.1, and the first human MHC II β polypeptide and the second human MHC II β polypeptide are each selected from the group consisting of a β chain of HLA-DR2, HLA-DR4, HLA-DQ2, HLA-DQ2.5, HLA-DQ8, and HLA-DQ8.1.

12. The method of claim 11, wherein the first endogenous MHC II locus and the second endogenous MHC II locus are modified successively in the same ES cell.

13. The method of claims 11 or 12, wherein the first mouse MHC II α polypeptide and the second mouse MHC II α polypeptide are each selected from the group consisting of an α chain of H-2A and H-2E and the first mouse MHC II β polypeptide and the second mouse MHC II β polypeptide are each selected from the group consisting of a β chain of H-2A and H-2E.

## Patentansprüche

1. Nagetier, das Folgendes umfasst:
(A) eine Nucleotidsequenz, die für ein erstes chimäres Mensch/Nagetier-MHC-II-α-Polypeptid kodiert, und eine Nucleotidsequenz, die für ein erstes chimäres Mensch/Nagetier-MHC-II-β-Polypeptid kodiert, an einem ersten endogenen MHC-II-Locus,
wobei das erste chimäre Mensch/Nagetier-MHC-II-α-Polypeptid α1- und α2-Domänen eines menschlichen HLA-DR-α-Polypeptids umfasst, die operabel an Transmembran- und Zytoplasma-Domänen eines endogenen Nagetier-MHC-II-α-Polypeptids gebunden sind,
wobei das erste chimäre Mensch/Nagetier-MHC-II-β-Polypeptid β1- und β2-Domänen eines menschlichen HLA-DR-β-Polypeptids umfasst, die operabel an Transmembran- und Zytoplasma-Domänen eines endogenen Nagetier-MHC-II-β-Polypeptids gebunden sind, und
wobei das erste chimäre Mensch/Nagetier-MHC-II-α-Polypeptid und das erste chimäre Mensch/Nagetier-MHC-II-β-Polypeptid einen ersten MHC-II-Komplex auf einer Oberfläche einer Zelle des Nagetiers bilden; und
(B) eine Nucleotidsequenz, die für ein zweites chimäres Mensch/Nagetier-MHC-II-α-Polypeptid kodiert, und eine Nucleotidsequenz, die für ein zweites chimäres Mensch/Nagetier-MHC-II-β-Polypeptid kodiert, an einem zweiten endogenen MHC-II-Locus,
wobei das zweite chimäre Mensch/Nagetier-MHC-II-α-Polypeptid α1- und α2-Domänen eines menschlichen HLA-DQ-α-Polypeptids umfasst, die operabel an Transmembran- und Zytoplasma-Domänen eines endogenen Nagetier-MHC-II-α-Polypeptids gebunden sind,
wobei das zweite chimäre Mensch/Nagetier-MHC-II-β-Polypeptid β1- und β2-Domänen eines menschlichen HLA-DQ-β-Polypeptids umfasst, die operabel an Transmembran- und Zytoplasma-Domänen eines endogenen Nagetier-MHC-II-β-Polypeptids gebunden sind,
wobei das zweite chimäre Mensch/Nagetier-MHC-II-α-Polypeptid und das zweite chimäre Mensch/Nagetier-MHC-II-β-Polypeptid einen zweiten MHC-II-Komplex auf einer Oberfläche einer Zelle des Nagetiers bilden und
wobei (i) das HLA-DR-α-Polypeptid ein HLA-DR2-α-Polypeptid ist und das HLA-DR-β-Polypeptid ein HLA-DR2-β-Polypeptid ist oder (ii) das HLA-DR-α-Polypeptid ein HLA-DR4-α-Polypeptid ist, das HLA-DR-β-Polypeptid ein HLA-DR4-β-Polypeptid ist, das HLA-DQ-α-Polypeptid ein HLA-DQ2- oder HLA-DQ8-α-Polypeptid ist und das HLA-DQ-β-Polypeptid ein HLA-DQ2- oder HLA-DQ8-β-Polypeptid ist.

2. Nagetier nach Anspruch 1, wobei die Nucleotidsequenz, die für das erste chimäre Mensch/Nagetier-MHC-II-α-Polypeptid kodiert, und die Nucleotidsequenz, die für das zweite chimäre Mensch/Nagetier-MHC-II-α-Polypeptid kodiert, operabel an einen endogenen Nagetier-MHC-II-α-Promotor und regulierende Elemente gebunden sind und wobei die Nucleotidsequenz, die für das erste chimäre Mensch/Nagetier-MHC-II-β-Polypeptid kodiert, und die Nucleotidsequenz, die für das zweite chimäre Mensch/Nagetier-MHC-II-β-Polypeptid kodiert, operabel an einen endogenen Nagetier-MHC-II-β-Promotor und regulierende Elemente gebunden sind.

3. Nagetier nach Anspruch 1, wobei das Nagetier kein funktionelles endogenes MHC-II-Polypeptid aus dem ersten endogenen MHC-II-Locus oder aus dem zweiten endogenen MHC-II-Locus exprimiert.

4. Nagetier nach einem der Ansprüche 1 bis 3 wobei das Nagetier eine Ratte ist.

5. Nagetier nach einem der Ansprüche 1 bis 3, wobei das Nagetier eine Maus ist.

6. Nagetier nach Anspruch 5, wobei der erste endogene MHC-II-Locus ein endogener H-2E-Locus ist und wobei das erste chimäre Mensch/Nagetier-MHC-II-α-Polypeptid die α1- und α2-Domänen eines menschlichen HLA-DR2- oder HLA-DR4-Polypeptids umfasst, die operabel an eine Transmembrandomäne und eine Zytoplasma-Domäne eines Maus-H-2E-α-Polypeptids gebunden sind, und wobei das erste chimäre Mensch/Nagetier-MHC-II-β-Polypeptid die β1- und β2-Domänen des menschlichen HLA-DR2- oder HLA-DR4-β-Polypeptids umfasst, die operabel an eine Transmembrandomäne und eine Zytoplasma-Domäne aus einem Maus-H-2E-β-Polypeptid gebunden sind.

7. Nagetier nach Anspruch 5, wobei der zweite endogene MHC-II-Locus ein endogener H-2A-Locus ist und wobei das zweite chimäre Mensch/Nagetier-MHC-II-α-Polypeptid die α1- und α2-Domänen eines menschlichen HLA-DQ2- oder HLA-DQ8-α-Polypeptids umfasst, die operabel an eine Transmembrandomäne und eine Zytoplasma-Domäne aus einem Maus-H-2A-α-Polypeptid gebunden sind, und wobei das zweite chimäre Mensch/Nagetier-MHC-II-β-Polypeptid die β1- und β2-Domänen eines menschlichen HLA-DQ2- oder HLA-DQ8-β-Polypeptids umfasst, die operabel an eine Transmembrandomäne und eine Zytoplasma-Domäne eines Maus-H-2A-β-Polypeptids gebunden sind.

8. Nagetier nach Anspruch 6, wobei dem endogenen H-2E-Locus eine endogene Nucleotidsequenz fehlt, die für α1- und α2-Domänen eines Maus-H-2E-α-Polypeptids kodiert, und wobei dem endogenen H-2E-Locus eine endogene Nucleotidsequenz fehlt, die für β1- und β2-Domänen eines Maus-H-2E-β-Polypeptids kodiert.

9. Nagetier nach Anspruch 7, wobei dem endogenen H-2A-Locus eine endogene Nucleotidsequenz fehlt, die für α1- und α2-Domänen eines Maus-H-2A-α-Polypeptids kodiert, und wobei dem endogenen H-2A-Locus eine endogene Nucleotidsequenz fehlt, die für β1- und β2-Domänen eines Maus-H-2A-β-Polypeptids kodiert.

10. Nagetier nach einem der Ansprüche 1 bis 3 oder der Ansprüche 5 bis 7, wobei das Nagetier eine Maus ist und wobei:
(a) der erste endogene MHC-II-Locus ein endogener MHC-II-H-2E-Locus ist, das erste chimäre Mensch/Nagetier-MHC-II-α-Polypeptid ein chimäres Mensch/Nagetier-DR2/H-2E-MHC-II-α-Polypeptid ist und das erste chimäre Mensch/Nagetier-MHC-II-β-Polypeptid ein chimäres Mensch/Nagetier-HLA-DR2/H-2E-MHC-II-β-Polypeptid ist; und
(b) der zweite endogene MHC-II-Locus ein endogener MHC-II-H-2A-Locus ist, das zweite chimäre Mensch/Nagetier-MHC-II-α-Polypeptid ein chimäres Mensch/Nagetier-DQ8.1/H-2A-MHC-II-α-Polypeptid ist und das zweite chimäre Mensch/Nagetier-MHC-II-β-Polypeptid ein chimäres Mensch/Nagetier-HLA-DQ8.1/H-2A-MHC-II-β-Polypeptid ist,
wobei die Maus einen chimären Mensch/Maus-HLA-DR2/H-2E-MHC-II-Komplex und einen chimären Mensch/Maus-HLA-DQ8.1/H-2A-MHC-II-Komplex auf einer Oberfläche einer Zelle der Maus exprimiert.

11. Verfahren zum Modifizieren von endogenen MHC-II-Loci in einer Maus-ES-Zelle, wobei das Verfahren Folgendes umfasst:
das Ersetzen von Folgendem an einem ersten endogenen MHC-II-Locus in der ES-Zelle:
zumindest einer Nucleotidsequenz, die für α1- und α2-Domänen eines ersten Maus-MHC-II-α-Polypeptids kodiert, durch zumindest eine Nucleotidsequenz, die für α1- und α2-Domänen eines ersten menschlichen MHC-II-α-Polypeptids kodiert, und
zumindest einer Nucleotidsequenz, die für β1- und β2-Domänen eines ersten Maus-MHC-II-β-Polypeptids kodiert, durch zumindest eine Nucleotidsequenz, die für β1- und β2-Domänen eines ersten menschlichen MHC-II-β-Polypeptids kodiert,
um einen ersten modifizierten chimären MHC-II-Locus zu erzeugen, der für α1- und α2-Domänen des ersten menschlichen MHC-II-α-Polypeptids kodiert, das operabel an Transmembran- und Zytoplasma-Domänen des ersten Maus-MHC-II-α-Polypeptids gebunden ist, und für β1- und β2-Domänen des ersten menschlichen MHC-II-β-Polypeptids kodiert, das operabel an Transmembran- und Zytoplasma-Domänen des ersten Maus-MHC-II-β-Polypeptids gebunden ist; und
das Ersetzen von Folgendem an einem zweiten endogenen MHC-II-Locus in der ES-Zelle:
zumindest einer Nucleotidsequenz, die für α1- und α2-Domänen eines zweiten Maus-MHC-II-α-Polypeptids kodiert, durch zumindest eine Nucleotidsequenz, die für α1- und α2-Domänen eines zweiten menschlichen MHC-II-α-Polypeptids kodiert, und
zumindest einer Nucleotidsequenz, die für β1- und β2-Domänen eines zweiten Maus-MHC-II-β-Polypeptids kodiert, durch zumindest eine Nucleotidsequenz, die für β1- und β2-Domänen eines zweiten menschlichen MHC-II-β-Polypeptids kodiert,
um einen zweiten modifizierten chimären MHC-II-Locus zu erzeugen, der für α1- und α2-Domänen des zweiten menschlichen MHC-II-α-Polypeptids kodiert, das operabel an Transmembran- und Zytoplasma-Domänen des zweiten Maus-MHC-II-α-Polypeptids gebunden ist, und für β1- und β2-Domänen des zweiten menschlichen MHC-II-β-Polypeptids kodiert, das operabel an Transmembran- und Zytoplasma-Domänen des zweiten Maus-MHC-II-β-Polypeptids gebunden ist; und
wobei das erste menschliche MHC-II-α-Polypeptid und das zweite menschliche MHC-II-α-Polypeptid jeweils aus der aus einer α-Kette von HLA-DR2, HLA-DR4, HLA-DQ2, HLA-DQ2.5, HLA-DQ8 und HLA-DQ8.1 bestehenden Gruppe ausgewählt sind und wobei das erste menschliche MHC-II-β-Polypeptid und das zweite menschliche MHC-II-β-Polypeptid jeweils aus der aus einer β-Kette von HLA-DR2, HLA-DR4, HLA-DQ2, HLA-DQ2.5, HLA-DQ8 und HLA-DQ8.1 bestehenden Gruppe ausgewählt sind.

12. Verfahren nach Anspruch 11, wobei der erste endogene MHC-II-Locus und der zweite endogene MHC-II-Locus nacheinander in derselben ES-Zelle modifiziert werden.

13. Verfahren nach Anspruch 11 oder 12, wobei das erste Maus-MHC-II-α-Polypeptid und das zweite Maus-MHC-II-α-Polypeptid jeweils aus der aus einer α-Kette von H-2A und H-2E bestehenden Gruppe ausgewählt sind und wobei das erste Maus-MHC-11-β-Polypeptid und das zweite Maus-MHC-11-β-Polypeptid jeweils aus der aus einer β-Kette von H-2A und H-2E bestehenden Gruppe ausgewählt sind.

## Revendications

1. Rongeur comprenant
(A) au niveau d'un premier locus de MHC II endogène, une séquence nucléotidique codant pour un premier polypeptide MHC II α d'humain/de rongeur chimérique et une séquence nucléotidique codant pour un premier polypeptide MHC II β d'humain/de rongeur chimérique,
dans lequel le premier polypeptide MHC II α d'humain/de rongeur chimérique comprend des domaines α1 et α2 d'un polypeptide HLA-DR α d'humain liés de manière opérationnelle à des domaines transmembranaires et cytoplasmiques d'un polypeptide MHC II α de rongeur endogène,
dans lequel le premier polypeptide MHC II β d'humain/de rongeur chimérique comprend des domaines β1 et β2 d'un polypeptide HLA-DR β d'humain liés de manière opérationnelle à des domaines transmembranaires et cytoplasmiques d'un polypeptide MHC II β de rongeur endogène, et
dans lequel le premier polypeptide MHC II α d'humain/de rongeur chimérique et le premier polypeptide MHC II β d'humain/de rongeur chimérique forment un premier complexe MHC II sur une surface d'une cellule du rongeur ; et
(B) au niveau d'un second locus de MHC II endogène, une séquence nucléotidique codant pour un second polypeptide MHC II α d'humain/de rongeur chimérique et une séquence nucléotidique codant pour un second polypeptide MHC II β d'humain/de rongeur chimérique,
dans lequel le second polypeptide MHC II α d'humain/de rongeur chimérique comprend des domaines α1 et α2 d'un polypeptide HLA-DQ α d'humain liés de manière opérationnelle à des domaines transmembranaires et cytoplasmiques d'un polypeptide MHC II α de rongeur endogène,
dans lequel le second polypeptide MHC II β d'humain/de rongeur chimérique comprend des domaines β1 et β2 d'un polypeptide HLA-DQ β d'humain liés de manière opérationnelle à des domaines transmembranaires et cytoplasmiques d'un polypeptide MHC II β de rongeur endogène,
dans lequel le second polypeptide MHC II α d'humain/de rongeur chimérique et le second polypeptide MHC II β d'humain/de rongeur chimérique forment un second complexe MHC II sur une surface d'une cellule du rongeur, et
dans lequel (i) le polypeptide HLA-DR α est un polypeptide HLA-DR2 α et le polypeptide HLA-DR β est un polypeptide HLA-DR2 β, ou (ii) le polypeptide HLA-DR α est un polypeptide HLA-DR4 α, le polypeptide HLA-DR β est un polypeptide HLA-DR4 β, le polypeptide HLA-DQ α est un polypeptide HLA-DQ2 ou HLA-DQ8 α et le polypeptide HLA-DQ β est un polypeptide HLA-DQ2 ou HLA-DQ8 β.

2. Rongeur selon la revendication 1, dans lequel la séquence nucléotidique codant pour le premier polypeptide MHC II α d'humain/de rongeur chimérique et la séquence nucléotidique codant pour le second polypeptide MHC II α d'humain/de rongeur chimérique sont liées de manière opérationnelle à un promoteur de MHC II α de rongeur endogène et à des éléments de régulation, et la séquence nucléotidique codant pour le premier polypeptide MHC II β d'humain/de rongeur chimérique et la séquence nucléotidique codant pour le second polypeptide MHC II β d'humain/de rongeur chimérique sont liées de manière opérationnelle à un promoteur de MHC II β de rongeur endogène.

3. Rongeur selon la revendication 1, dans lequel le rongeur n'exprime pas un polypeptide MHC II endogène fonctionnel à partir du premier locus de MHC II endogène ou à partir du second locus de MHC II endogène.

4. Procédé selon l'une quelconque des revendications 1 à 3, dans lequel le rongeur est un rat.

5. Procédé selon l'une quelconque des revendications 1 à 3, dans lequel le rongeur est une souris.

6. Rongeur selon la revendication 5, dans lequel le premier locus de CMH II endogène est un locus de H-2E endogène et dans lequel le premier polypeptide MHC II α d'humain/de rongeur chimérique comprend les domaines α1 et α2 d'un polypeptide HLA-DR2 ou HLA-DR4 humain liés de manière opérationnelle à un domaine transmembranaire et à un domaine cytoplasmique d'un polypeptide H-2E α de souris et le premier polypeptide d'humain/ de rongeur chimérique CMH II β comprend les domaines β1 et β2 du polypeptide HLA-DR2 ou HLA-DR4 β humain liés de manière opérationnelle à un domaine transmembranaire et à un domaine cytoplasmique d'un polypeptide H-2E β de souris.

7. Rongeur selon la revendication 5, dans lequel le second locus de MHC II endogène est un locus de H-2A endogène et dans lequel le second polypeptide MHC II α d'humain/de rongeur chimérique comprend les domaines α1 et α2 d'un polypeptide HLA-DQ2 ou HLA-DQ8 α humain liés de manière opérationnelle à un domaine transmembranaire et à un domaine cytoplasmique d'un polypeptide H-2A α de souris et le second polypeptide MHC II β d'humain/de rongeur chimérique comprend les domaines β1 et β2 d'un polypeptide HLA-DQ2 ou HLA-DQ8 β humain liés de manière opérationnelle à un domaine transmembranaire et à un domaine cytoplasmique d'un polypeptide H-2A β de souris.

8. Rongeur selon la revendication 6, dans lequel le locus de H-2E endogène est dépourvu d'une séquence nucléotidique endogène codant pour des domaines α1 et α2 d'un polypeptide H-2E α de souris, et le locus H-2E endogène est dépourvu d'une séquence nucléotidique endogène codant pour des domaines β1 et β2 d'un polypeptide H-2E β de souris.

9. Rongeur selon la revendication 7, dans lequel le locus de H-2A endogène est dépourvu d'une séquence nucléotidique endogène codant pour des domaines α1 et α2 d'un polypeptide H-2A α de souris, et le locus H-2A endogène est dépourvu d'une séquence nucléotidique endogène codant pour des domaines β1 et β2 d'un polypeptide H-2A β de souris.

10. Rongeur selon la revendication 1 à 3 ou des revendication 5 à 7, dans lequel le rongeur est une souris, et dans lequel :
(a) le premier locus de MHC II endogène est un locus de MHC II H-2E endogène, le premier polypeptide MHC II α d'humain/de rongeur chimérique est un polypeptide HLA-DR2/H-2E MHC II α d'humain/de rongeur chimérique et le premier polypeptide MHC II β d'humain/de rongeur chimérique est un polypeptide HLA-DR2/H-2E MHC II β d'humain/de rongeur chimérique ; et
(b) le second locus de MHC II endogène est un locus de MHC II H-2A endogène, le second polypeptide MHC II α d'humain/de rongeur chimérique est un polypeptide MHC II α d'humain/de rongeur chimérique HLA-DQ8.1/H-2A MHC II α et le second polypeptide MHC II β d'humain/de rongeur chimérique est un polypeptide HLA-DQ8.1/H-2A MHC II β d'humain/de rongeur chimérique,
dans lequel la souris exprime un complexe de HLA-DR2/H-2E MHC II d'humain/de rongeur chimérique et un complexe HLA-DQ8.1/H-2A MHC II d'humain/de souris chimérique sur une surface d'une cellule de la souris.

11. Procédé de modification de loci de MHC II endogène dans une cellule ES de souris, dans lequel le procédé comprend les étapes consistant à :
remplacer au niveau d'un premier locus de MHC II endogène dans la cellule ES :
au moins une séquence nucléotidique codant pour des domaines α1 et α2 d'un premier polypeptide MHC II α de souris avec au moins une séquence nucléotidique codant pour des domaines α1 et α2 d'un premier polypeptide de MHC II α d'humain, et
au moins une séquence nucléotidique codant pour des domaines β1 et β2 d'un premier polypeptide MHC II β de souris avec au moins une séquence nucléotidique codant pour des domaines β1 et β2 d'un premier polypeptide MHC II β d'humain,
pour générer un premier locus de MHC II chimérique modifié codant pour des domaines α1 et α2 du premier polypeptide MHC II α d'humain liés de manière opérationnelle à des domaines transmembranaires et cytoplasmiques du premier polypeptide MHC II α de souris et des domaines β1 et β2 du premier polypeptide de MHC II β d'humain liés de manière opérationnelle à des domaines transmembranaires et cytoplasmiques du premier polypeptide MHC II β de souris ; et
remplacer au niveau d'un second locus de MHC II endogène dans la cellule ES :
au moins une séquence nucléotidique codant pour des domaines α1 et α2 d'un second polypeptide MHC II α de souris avec au moins une séquence nucléotidique codant pour des domaines α1 et α2 d'un second polypeptide MHC II α d'humain, et
au moins une séquence nucléotidique codant pour des domaines β1 et β2 d'un second polypeptide MHC II β de souris avec au moins une séquence nucléotidique codant pour des domaines β1 et β2 d'un second polypeptide MHC II β d'humain,
pour générer un second locus de MHC II chimérique modifié codant des domaines α1 et α2 du second polypeptide MHC II α d'humain liés de manière opérationnelle aux domaines transmembranaires et cytoplasmiques du second polypeptide MHC II α de souris et des domaines β1 et β2 du second polypeptide de MHC II β d'humain liés de manière opérationnelle aux domaines transmembranaires et cytoplasmiques du second polypeptide MHC II β de souris, et
dans lequel le premier polypeptide MHC II α d'humain et le second polypeptide MHC II α d'humain sont chacun choisis dans le groupe constitué d'une chaîne α de HLA-DR2, de HLA-DR4, de HLA-DQ2, de HLA-DQ2.5, de HLA-DQ8 et de HLA-DQ8.1, et le premier polypeptide MHC II β d'humain et le second polypeptide MHC II β d'humain sont chacun choisis dans le groupe constitué d'une chaîne β de HLA-DR2, de HLA-DR4, de HLA-DQ2, de HLA-DQ2.5, de HLA-DQ8 et de HLA-DQ8.1.

12. Procédé selon la revendication 11, dans lequel le premier locus de MHC II endogène et le second locus de MHC II endogène sont modifiés successivement dans la même cellule ES.

13. Procédé selon la revendication 11 à 12, dans lequel le premier polypeptide MHC II α de souris et le second polypeptide MHC II α de souris sont chacun choisis dans le groupe constitué d'une chaîne α de H-2A et de H-2E et le premier polypeptide MHC II β de souris et le second polypeptide MHC II β de souris sont chacun choisis dans le groupe constitué d'une chaîne β de H-2A et de H-2E.
